(19) Europäisches Patentamt
European Patent Office
Office européen des brevets

(11) EP 2 916 859 B1

(12) EUROPEAN PATENT SPECIFICATION

(45) Date of publication and mention of the grant of the patent:
**28.06.2017 Bulletin 2017/26**

(21) Application number: **13792162.3**

(22) Date of filing: **01.11.2013**

(51) Int Cl.:
***A61K 38/21*** *(2006.01)* ***A61P 27/00*** *(2006.01)*

(86) International application number:
**PCT/US2013/068056**

(87) International publication number:
**WO 2014/071183 (08.05.2014 Gazette 2014/19)**

# (54) METHOD OF REDUCING ADVERSE EFFECTS IN A CANCER PATIENT UNDERGOING TREATMENT WITH A MEK INHIBITOR

VERFAHREN ZUR REDUKTION VON NEBENWIRKUNGEN BEI EINEM MIT EINEM MEK-HEMMER BEHANDELTEN KREBSPATIENTEN

MÉTHODE DE RÉDUCTION DES EFFETS SECONDAIRES CHEZ UN PATIENT SOUFFRANT DE CANCER TRAITÉ PAR UN INHIBITEUR DE LA MEK

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.11.2012 US 201261721810 P**

(43) Date of publication of application:
**16.09.2015 Bulletin 2015/38**

(73) Proprietors:
- **The U.S.A. as represented by the Secretary, Department of Health and Human Services Bethesda, MD 20892-7660 (US)**
- **Merck Patent GmbH 64293 Darmstadt (DE)**

(72) Inventors:
- **MILLER, Sheldon S. Bethesda, MD 20817 (US)**
- **MAMINISHKIS, Arvydas Washington, D.C. 20009 (US)**
- **REMÉ, Charlotte E. CH-8044 Zürich (CH)**

(74) Representative: **Carpmaels & Ransford LLP One Southampton Row London WC1B 5HA (GB)**

(56) References cited:
- **MAMINISHKIS ET AL: "IFN gamma regulates pigment epithelial fluid transport.", AM.J.CELL.PHYSIOL., vol. 297, 30 September 2009 (2009-09-30), - 30 September 2009 (2009-09-30), pages 1452-1465, XP002718368,**
- **HAURA ET AL: "Phase II study of PD-0325901, and oral MEK inhibitor in previoulsy treated patients with Advanced no small cell lung cancer", CLIN.CAN.RES., vol. 16, 23 March 2010 (2010-03-23), - 23 March 2010 (2010-03-23), pages 2450-2457, XP002718369,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present description is directed to a pharmaceutical composition comprising IFNγ for use in a method for treating an adverse side effect of MEK inhibitors by administering a pharmaceutical composition comprising IFNγ to a cancer patient being treated by a MEK-inhibitor.

BACKGROUND

Cancer is the leading cause of death in United States, Europe, and Japan. The overall five year survival rate for all cancers is only 65%. There continues to be a great unmet need for cancer therapies.

The Ras/Raf/MEK/ERK cascade is one of the major pathways transmitting signals from the cell surface to the nucleus. Rat sarcoma (Ras) activation through the Raf/MEK/ERK pathway modulates the activity of nuclear factors that regulate the transcription of genes that are required for proliferation and differentiation and that modulate the cell cycle. The extracellular signal-regulated kinases (ERK) pathway is implicated in mechanisms of cell survival and apoptosis.

Activating Ras mutations are found in 30% of cancers. This mutation is found in high incidence in several tumor types: 90% of pancreatic, 45% of colonic, 60% of thyroid, 35% of small-cell lung cancer, as well as in acute myeloid leukemia (AML) and acute lymphobastic leukemia (ALL). Ras mutations are associated with resistance to epidermal growth factor receptor (EGFR) tyrosine kinase inhibitors, such as gefitinib, erlotinib, panitumumab, and cetuximab. MAP kinase/ERK kinase (MEK), an oncogene or signal protein within the P38 mitogen activated protein kinase (MAPK) pathway, is a crucial point of convergence that integrates a variety of protein kinases through Ras. The MEK pathway is important for malignant transformation and progression of certain tumor types. For example, a MEK1 mutation has been identified in primary lung tumors (NSCLC). Activation of this pathway likely is sufficient to drive tumor progression and malignancy.

Inhibitors of MEK are used to treat a variety of proliferative diseases, including cancers, because of their anti-angiogenic and/or vascular permeability reducing effect. MEK inhibitors may be used alone and in various combination therapies with other chemotherapeutic drugs. MEK inhibitors are currently being tested in monotherapies and combination therapies against a wide variety of cancers in a large number of Phase I and Phase II clinical trials, including RAI-refractory metastatic thyroid cancer, colorectal cancer, multiple myeloma, hepatocellular carcinoma, glioma, cancers with BRAF mutations, melanoma, solid tumors and soft tissue sarcoma, biliary track cancer, NSCLC, pancreatic cancer, liver cancer, breast cancer, lymphoma, and leukemia (NCI/NIH, Clinical Trials Index at www.cancer.gov; Trujillo, 2011, Expert Opin Ther Patents 21:1045-69).

A variety of side effects are associated with treatment of cancer with MEK inhibitors, including visual disturbances. The visual disturbances associated with MEK inhibitors included blurred vision, halos, spots, and decreased acuity (Messersmith et al., 2006, Clin Adv Hematol Oncol 4:831-36; Haura et al. 2010, Clin Cancer Res 16:2450-57; Renouf et al., 2011, JCO 41:5851; Fremin et al. 2010, J Hemotol Oncol 3:8-11). For example, retinal vascular thrombosis, retinal vascular disorder and retinopathy were reported to the FDA in connection with the use of MEK inhibitor dihydroxypropyl fluoroethoxyphen cyclopropa sulfamide (DrugCite 2012). Ocular adverse events have been reported in connection with administration of pimasertib (Girard et al., PAGE 2012 meeting on 8 June 2012, Venice, Italy). Other severe side effects are reported to occur from administration of MEK inhibitor. These include serious skin rashes, diarrhea, asthenia, and dangerous drops in white blood cell counts (NCI Cancer Research Updates, June 24, 2013; Haura et al., 2010, Clin Cancer Res 16:2450-57; Rinehart et al. 2004, J Clin Oncol 22:4456-62; Wang et al., 2007, BBA Mol Cell Res 1773:1248-55).

These adverse events cause discontinued use of MEK inhibitors in affected patients who are withdrawn from therapy and the patient's cancer left untreated, ultimately affecting the affected patient's survival.

There remains an unmet need for treating the side effects associated with this class of cancer therapeutics. In particular, there is a need to develop coordinately administered therapies that treat the adverse side effects of MEK inhibitors, an emerging class of therapeutics for treating cancer.

SUMMARY

One aspect of the description is a MEK inhibitor and a pharmaceutical composition comprising IFNγ for use in a method for treating a cancer patient comprising administering a MEK inhibitor and a pharmaceutical composition comprising IFNγ. In an embodiment of the method, the MEK inhibitor may be a compound selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766. Preferably, the method uses pimasertib as the MEK inhibitor. In another embodiment of the method, the MEK inhibitor can be co-administered with

a chemotherapeutic agent selected from the group consisting of a dual inhibitor of phosphatidylinositol-3-kinase/mammalian target of rapamycin kinases (PI3K/mTOR) pathways, a P13K inhibitor, an antimetabolite, an antimitotic agent, an inhibitor of an epidermal growth factor receptor (EGFR), a tyrosine kinase inhibitor, an alkylating agent, and an inhibitor of B-raf protein kinase (BRAF). In another embodiment, the method further involves administering a chemotherapeutic agent selected from the group consisting of SAR245409, AZD8055, temsirolimus, everolimus, BEZ235, SAR2455408, GDC-0941, fluorouracil, capecitabine, gemcitabine, FOLFIRI, docetaxel, paclitaxel, pemetrexed, cetuximab, imatinib, erlotinib, gefitinib, dacarbazine, tomozolomide, dabrafenib, RO5212054, ARQ 736, and vemurafenib. In a preferred embodiment, the method consists of administering pimasertib and a P13K inhibitor, more preferably SAR245409 or SAR2455408. The method may be applied for the cancer patient who has a solid tumor, a hematological malignancy, a pancreatic cancer, or a colorectal cancer. Preferably, the cancer patient is one having a visual disturbance induced by administration of the MEK inhibitor, more preferably a patient having a retinal detachment, most preferably one in which the retinal detachment is caused by an increase in the amount of fluid present in the retina and/or subretinal space. In a further embodiment of the method, the pharmaceutical composition comprising IFNγ is administered in an amount effective to treat the visual disturbance, or in an amount to decrease the fluid present in the retina and/or subretinal space of the cancer patient. In another embodiment of the method, the pharmaceutical composition comprising IFNγ is administered to the basolateral side of the retinal pigment epithelium, preferably via a liquid droplet administered to the anterior surface of the eye.

Another aspect of the description is a pharmaceutical composition comprising IFNγ for use in a method for treating a visual disturbance caused by administration of a MEK inhibitor to a cancer patient, comprising administering of a pharmaceutical composition comprising IFNγ. The MEK inhibitor may be a compound selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766, and optionally may be co-administered with a chemotherapeutic agent selected from the group consisting of a dual inhibitor of phosphatidylinositol-3-kinase/mammalian target of PI3K/mTOR pathways, a P13K inhibitor, an antimetabolite, an antimitotic agent, an inhibitor of an EGFR, a tyrosine kinase inhibitor, an alkylating agent, and an inhibitor of BRAF, for example SAR245409, AZD8055, temsirolimus, everolimus, BEZ235, SAR2455408, GDC-0941, fluorouracil, capecitabine, gemcitabine, FOLFIRI, docetaxel, paclitaxel, pemetrexed, cetuximab, imatinib, erlotinib, gefitinib, dacarbazine, tomozolomide, dabrafenib, RO5212054, ARQ 736, or vemurafenib. In this embodiment of the description, preferably, the cancer patient is one in which the MEK inhibitor induces a visual disturbance, more preferably one in which the visual disturbance is caused by retinal detachment, most preferably one in which the retinal detachment is caused by an increase the amount of fluid present in the retina and/or subretinal space. In a further embodiment of the method the pharmaceutical composition comprising IFNγ is administered in an amount effective to treat the visual disturbance, preferably in an amount to decrease the fluid present in the retina and/or subretinal space of the cancer patient. In another embodiment of the method, the pharmaceutical composition comprising IFNγ is administered to the basolateral side of the retinal pigment epithelium, preferably via a liquid droplet administered to the anterior surface of the eye.

Another aspect of the description is a pharmaceutical composition comprising IFNγ for use in a method for treating an adverse event caused by administration of a MEK inhibitor to a cancer patient, comprising administering of a pharmaceutical composition comprising IFNγ. The MEK inhibitor may be a compound selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766, and optionally may be co-administered with a chemotherapeutic agent selected from the group consisting of a dual inhibitor of phosphatidylinositol-3-kinase/mammalian target of PI3K/mTOR pathways, a P13K inhibitor, an antimetabolite, an antimitotic agent, an inhibitor of an EGFR, a tyrosine kinase inhibitor, an alkylating agent, and an inhibitor of BRAF, for example SAR245409, AZD8055, temsirolimus, everolimus, BEZ235, SAR2455408, GDC-0941, fluorouracil, capecitabine, gemcitabine, FOLFIRI, docetaxel, paclitaxel, pemetrexed, cetuximab, imatinib, erlotinib, gefitinib, dacarbazine, tomozolomide, dabrafenib, RO5212054, ARQ 736, or vemurafenib. In this embodiment of the description, preferably, the cancer patient is one in which the MEK inhibitor induces skin rashes, diarrhea, asthenia, or severe drops in white blood cell counts. In a further embodiment of the method the pharmaceutical composition comprising IFNγ is administered in an amount effective to treat the adverse event.

Also described herein is a use of a MEK inhibitor for the manufacture of a medicament for treating a cancer patient by administering the medicament and a pharmaceutical composition comprising IFNγ. In the use, the medicament may be a MEK inhibitor selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766, or a combination of a MEK inhibitor with a chemotherapeutic agent selected from the group consisting of a dual inhibitor of phosphatidylinositol-3-kinase/mammalian target of rapamycin kinases (PI3K/mTOR) pathways, a P13K inhibitor, an antimetabolite, an antimitotic agent, an inhibitor of an epidermal growth factor receptor (EGFR), a tyrosine kinase inhibitor, an alkylating agent, or an inhibitor of B-raf protein kinase (BRAF), for example, SAR245409, AZD8055, temsirolimus, everolimus, BEZ235, SAR2455408, GDC-0941, fluorouracil, capecitabine, gemcitabine, FOLFIRI, docetaxel, paclitaxel, pemetrexed, cetuximab, imatinib, erlotinib, gefitinib, dacarbazine, tomozolomide, dabrafenib, RO5212054, ARQ 736, or vemurafenib. The use of the medicament can be directed to cancer patients who have a solid tumor, a hematological malignancy, a pancreatic cancer, or a colorectal cancer. The pharmaceutical composition comprising IFNγ is administered when the medicament induces a visual disturbance in the cancer patient, specifically, when the visual disturbance is caused by retinal detachment, more specifically when the retinal detachment is caused by an increase the amount of fluid present in the retina and/or subretinal space of the cancer patient. In such instance, the use of the description is directed to administering the pharmaceutical composition comprising IFNγ in an amount effective to treat the visual disturbance, for example, in an amount to decrease the fluid present in the retina and/or subretinal space of the cancer patient. Accordingly, the pharmaceutical composition comprising IFNγ is administered to the basolateral side of the retinal pigment epithelium, preferably to the anterior surface of the eye, for example in a liquid droplet.

Another aspect of the description is a MEK inhibitor for use in a method of treating a cancer patient, wherein the method comprises administering the MEK inhibitor and a pharmaceutical composition comprising IFNγ. The MEK inhibitor may consist of a compound selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766. The MEK inhibitor may be co-administered with a chemotherapeutic agent selected from the group consisting of a dual inhibitor of phosphatidylinositol-3-kinase/mammalian target of PI3K/mTOR pathways, a P13K inhibitor, an antimetabolite, an antimitotic agent, an inhibitor of an EGFR, a tyrosine kinase inhibitor, an alkylating agent, and an inhibitor of BRAF, for example, SAR245409, AZD8055, temsirolimus, everolimus, BEZ235, SAR2455408, GDC-0941, fluorouracil, capecitabine, gemcitabine, FOLFIRI, docetaxel, paclitaxel, pemetrexed, cetuximab, imatinib, erlotinib, gefitinib, dacarbazine, tomozolomide, dabrafenib, RO5212054, ARQ 736, or vemurafenib. The MEK inhibitor of this aspect of the description is administered to treat a cancer patient with a solid tumor, a hematological malignancy, a pancreatic cancer, or a colorectal cancer. The pharmaceutical composition comprising he IFNγ composition of this aspect of the description is administered when the MEK inhibitor induces a visual disturbance in the cancer patient, specifically, when the visual disturbance is caused by retinal detachment, more specifically when the retinal detachment is caused by an increase the amount of fluid present in the retina and/or subretinal space of the cancer patient. In such instance, the use of the description is directed to administering the pharmaceutical composition comprising IFNγ in an amount effective to treat the visual disturbance, for example, in an amount to decrease the fluid present in the retina and/or subretinal space of the cancer patient. In another embodiment of the method, the pharmaceutical composition comprising IFNγ is administered to the basolateral side of the retinal pigment epithelium, preferably via a liquid droplet administered to the anterior surface of the eye.

Another aspect of the description is a pharmaceutical composition comprising IFNγ for use in a method of treating a cancer patient, wherein the method comprises administering a MEK inhibitor and said pharmaceutical composition, for example a method in which the cancer patient has a solid tumor, a hematological malignancy, a pancreatic cancer, or a colorectal cancer. The pharmaceutical composition comprising IFNγ of this aspect of the description is administered when the MEK inhibitor induces a visual disturbance in the cancer patient, specifically, when the visual disturbance is caused by retinal detachment, more specifically when the retinal detachment is caused by an increase the amount of fluid present in the retina and/or subretinal space of the cancer patient. In such instance, the use of the description is directed to administering the pharmaceutical composition comprising IFNγ in an amount effective to treat the visual disturbance, for example, in an amount to decrease the fluid present in the retina and/or subretinal space of the cancer patient. In another embodiment of the method, the pharmaceutical composition comprising IFNγ is administered to the basolateral side of the retinal pigment epithelium, preferably via a liquid droplet administered to the anterior surface of the eye. In this aspect of the description, the pharmaceutical composition comprising IFNγ is administered when the MEK inhibitor comprises a compound selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766, in which the MEK inhibitor is administered alone or in combination with a chemotherapeutic agent selected from the group consisting of a dual inhibitor of phosphatidylinositol-3-kinase/mammalian target of PI3K/mTOR pathways, a P13K inhibitor, an antimetabolite, an antimitotic agent, an inhibitor of an EGFR, a tyrosine kinase inhibitor, an alkylating agent, and an inhibitor of BRAF, e.g., SAR245409, AZD8055, temsirolimus, everolimus, BEZ235, SAR2455408, GDC-0941, fluorouracil, capecitabine, gemcitabine, FOLFIRI, docetaxel, paclitaxel, pemetrexed, cetuximab, imatinib, erlotinib, gefitinib, dacarbazine, tomozolomide, dabrafenib, RO5212054, ARQ 736, or vemurafenib.

## BRIEF DESCRIPTION OF THE DRAWINGS

Fig. 1 shows the apical and basolateral membranes of the RPE represented as an equivalent electromotive force

(EMF), $E_A$ or $E_B$, in series with $R_A$ or $R_B$, respectively. The paracellular pathway is represented as a shunt resistor, $R_S$, which is the parallel combination of the junctional complex resistances between neighboring cells and the resistance caused by the seal around the circumference of the tissue.

Fig. 2A-2C show the effects of MEK inhibitor on transepithelial potential (TEP, --, in mV) and transepithelial resistance (TER, $R_T$, ◇, in $\Omega \cdot cm^{-2}$) of hfRPE cells following acute addition of the drug to the apical (Ap) or basolateral (Ba) bath of the Üssing electrophysiology chamber under continuous perfusion conditions. Time is on the horizontal axis, and five minute reference bar (5 min) is shown. Fig. 2A shows the effect of 10 μM pimasertib (MEK inhibitor), Fig. 2B shows the effect of 100 μM pimasertib, and Fig. 2C shows the effect of 200 μM pimasertib.

Fig. 3 shows the effect of chronic MEK inhibitor pretreatment of hfRPE cells on their responses to acute MEK inhibitor or ATP. The effect of pimasertib (MEK inhibitor) measured as shown in Figs. 2A-2C. The effect of acute addition of MEK inhibitor (200 μM MEK inhibitor added to both apical and basal chambers) on the viability of the hfRPE cells was tested by measuring the response of the cells to ATP added to the apical chamber.

Figs. 4A shows that DMSO (MEK inhibitor solubilizing agent) did not alter MEK inhibitor and ATP responses. Fig. 4B shows that 72 hr incubation with MEK inhibitor (10 μM) significantly reduced the cells response to acute MEK inhibitor or ATP.

Figs. 5A and 5B show the effect of chronic incubation with pimasertib (MEK inhibitor). The effect of MEK inhibitor measured as shown in Figs. 2A-2C. Cells were pretreated for 72 hours with 10 μM MEK inhibitor, and responses of cells to acute addition of 100 μM MEK inhibitor were measured. Fig. 5A shows the effect of MEK inhibitor on transepithelial potential (TEP, mV). Fig. 5B shows the effect on transepithelial resistance ($R_T$, $\Omega \cdot cm^{-2}$).

Figs. 6A-6C summarizes TEP and TER data for chronic MEK inhibitor pretreatment of hfRPE. Fig. 6A shows the effect of pretreatment with 50 μM, Fig. 6B shows the effect of 10 μM, and Fig. 6C shows the effect of 1 μM MEK inhibitor. Jv is a measure of fluid transport.

Fig. 7 shows that addition of IFNγ to the basal side of hfRPE can significantly increase fluid transport across RPE and that this increase can be blocked by CFTR inhibitors in the basal bath.

Fig. 8 shows that acute apical addition of 100 μM MEK inhibitor to hfRPE monolayers treated chronically (72 hours) with MEK inhibitor (10 μM) decreased fluid adsorption in response to IFNγ. Transepithelial fluid transport (Jv) was measured (upper panel, in $\mu l \cdot cm\text{-}2 \cdot hr^{-1}$) and is plotted as a function of time so that net fluid absorption (apical to basal bath) is indicated by positive values.

Fig. 9 shows the effect of chronic MEK inhibitor pretreatment of hfRPE on the response to ATP. Cells were pretreated with 10 μM MEK inhibitor for 72 hours. The effect of MEK inhibitor (100 μM) and ATP are shown to be significantly decreased.

Figs. 10A and 10B summarizes the result for chronic 72 hours treatment with 10 μM MEK inhibitor. The responses show that MEK inhibitor treatment reversed fluid transport. Fig. 10A shows the effect on secreting tissues. Fig. 10B shows the effect on absorbing tissues, and the rescue of fluid transport affected by addition of 15 ng/ml IFNγ.

Figs. 11A and 11B summarizes the result for acute addition of MEK inhibitor to apical bath bathing hfRPE. Fig. 11A shows the effect of 100 μM pimasertib and Fig. 11B shows the effect of 1 μM pimasertib. The effect of apical MEK inhibitor is reverse by basolateral 10 ng/ml IFNγ.

Fig. 12 shows a representation of some of the pathways for control of fluid transport in RPE cells.

## DETAILED DESCRIPTION OF ILLUSTRATIVE EMBODIMENTS

Applicants have discovered that MEK inhibitors decrease fluid transport from the retina and/or subretinal space to the serosal side of the retinal pigment epithelium (RPE). This results in accumulation of fluid in the retina and subretinal space, which causes retinal detachment. Similarly, other adverse events associated with treatment with MEK inhibitor, including skin rashes, diarrhea, asthenia, or severe drops in white blood cell counts, may be associated or caused by disruption of fluid transport across epithelial cell boundaries between serosal and basal layers. Applicants have also found that fluid transport is restored by treatment with IFNγ, which reverses the effect of MEK inhibitors.

RPE is a highly specialized derivative of the neuroectoderm with multiple roles in the maintenance of normal ocular function. The RPE is single monolayer of epithelial cells, located in the back of the vertebrate eye, between the choroidal blood supply (choriocapillaris) and the neuroretina. The RPE acts as one of the components of the blood-retinal barrier, and RPE cells play vital roles in maintaining the visual cycle, in photoreceptor outer segment phagocytosis, and in transport of nutrients, metabolic waste products, ions, and fluid between the distal retina and the choriocapillaris. Dysfunction of RPE cells has been implicated in inflammatory and degenerative diseases of the retina and choroid but relatively little is understood regarding the direct effects of inflammatory mediators on RPE physiology or pathophysiology.

In the eye, IFNγ plays important roles in macrophage activation and in the recruitment of inflammatory cells to sites of inflammation, and has been detected in vitreous aspirates of patients with uveitis, proliferative vitreoretinopathy, and other inflammatory eye diseases.

Interaction of IFNγ with its cell surface receptor on RPE cells activates receptor-associated Janus-activated

kinase 1 (JAK1) and JAK2, which in turn phosphorylate and activate the signal transducer and activator of transcription-1α (STAT-1α). Phosphorylated START-1α dimerizes and translocates into the nucleus where it binds to well-defined DNA sequences called IFNγ activation sites (GASs) in IFNγ-inducible promoters and activates the transcription of genes that encode members of the interferon regulatory factor (IRF) family of transcription factors (see Fig. 12).

Administration of IFNγ to the basolateral side of the RPE increases fluid transport ($J_V$) across the RPE, resulting in the absorption of fluid from the retina and/or subretinal space whether added acutely or chronically and can be administered by application to the anterior surface of the eye for the treatment of adverse ocular conditions in which subretinal fluid accumulation occurs.

Described herein is a method for treating a cancer, the method comprising administering a MEK inhibitor and a pharmaceutical formulation comprising IFNγ to a cancer patient. Also provided herein is a description of a method for treating adverse events caused by MEK inhibitors, including a visual disturbance caused by administration of a MEK inhibitor to a cancer patient, comprising administering a pharmaceutical formulation comprising IFNγ. Also described herein is a use of a MEK inhibitor for the manufacture of a medicament for treating a cancer, wherein the treatment comprises administering the medicament and a pharmaceutical formulation comprising IFNγ. Also described herein is a MEK inhibitor for use in a method for treating a cancer patient, the method comprising administering the MEK inhibitor and a pharmaceutical formulation comprising IFNγ. Also provided herein is a description of a pharmaceutical formulation comprising IFNγ for use in a method of treating a cancer patient, the method comprising administering a MEK inhibitor and said IFNγ formulation.

## Definitions

As used herein "visual disturbances" refers to one or more of the following: any decrease in visual acuity; blurred vision, halos, spots, retinal vascular thrombosis, retinal vascular disorder, or retinopathy.

As used herein, the phrase "decrease in visual acuity" refers to any diminishing or lessening of the acuteness or clearness of vision, and can refer to any measurable diminishing or lessening in the acuteness or clearness of form vision, which is dependent on the sharpness of the retinal focus within the eye and the sensitivity of the interpretative faculty of the brain.

As used herein, the phrase "accumulation of fluid in the retina and/or subretinal space" refers to an increase in the amount of fluid present in the space that separates the retinal pigment epithelium (RPE) from the outer segments of the photoreceptors beyond the amount of fluid normally present in that space in healthy eyes. The phrase "decrease the amount of fluid present in the retina and/or subretinal space," and all variations thereof, refers to any lessening or diminishing of the amount of fluid present in the space that separates the RPE from the outer segments of the photoreceptors.

As used herein, the phrase "basolateral side of the retinal pigment epithelium" refers to the side of the retinal pigment epithelium that is adjacent to, borders, or faces, the choroid.

As used herein, the phrase "anterior surface of the eye" refers to portion of the cornea that comprises the exterior, exposed part of the eye.

As used herein, the phrase "subretinal injection" refers to the introduction by any means of a substance into the subretinal space.

As used herein, the phrase "subtenon injection" refers to the introduction by any means of a substance into the area below the Tenon's capsule and above the sclera of the eye at a point posterior to a limbus of the eye.

As used herein, "IFNγ" refers to interferon-γ or, equivalently, interferon-γ1b (National Library of Medicine CAS number 82115-62-6; WHO ATC code L03AB03; NCBI Reference Sequence: NM_000619.2). Interferon gamma (IFNγ) is a pleiotropic cytokine produced by T- and NK- cells and is involved in the regulation of both innate and adaptive immune responses. The major biological activities of IFNγ are associated with antiviral and immunomodulatory effects, cell grow and differentiation, and control of apoptosis (Stark, G., et al., Annu Rev Biochem, 1998, 67, 227-264; Ramana, C., et al., Trends Immunol, 2002, 23, 96-101; van Boxel-Dezaire, A., et al., Curr Top Microbiol Immunol, 2007, 316, 119-154). The IFNγ receptor is composed of two distinct subunits, IFNGR1 and IFNGR2, in which IFNGR1 is the major ligand-binding subunit, (Stark, G., et al., Annu Rev Biochem, 1998, 67, 227-264; Bach, E., et al., Annu Rev Immunol, 1997, 15, 563-591) while IFNGR2 plays a critical role in the generation of IFNγ signals (Hemmi, S., et al., Cell, 1994, 76, 803-810; Soh, J., et al., Cell, 1994, 76, 793-802).

As used herein, "a MEK inhibitor" refers to an inhibitor of MAP kinase/ERK kinase (MEK), a protein kinase downstream of BRAF in the RAS/RAF/MAPK/ERK pathway. MEK inhibitors include pimasertib (MSC1936369B; AS703026; N-(2,3-dihydroxypropyl)-1-((2-fluoro-4-iodophenyl)amino)isonicotinamide)(Merck Sharp & Dohme/Ares-Serono), selumetinib (ARRY142886/AZD6244)(Array BioPharm/AstraZeneca), AZD8330 (AstraZeneca), GSK1120212 (GlaxoSmithKline), GDC0973 (Genentech), GDC0941, GDC0973/XL518 (Genentech/Elexis), CI1040/PD184352 (Pfizer), PD035901 (Pfizer), ARRY438162 (Array BioPharm), RDEA436 (Ardea), TAK733 (Takeda), R05126766 (Roche), and RDEA119/BAY869766 (Ardea/Bayer).

As used herein a "combination therapy with a MEK inhibitor" refers to a chemotherapeutic agent used in combination with a MEK inhibitor. The chemotherapeutic agent may be a dual inhibitor of phosphatidylinositol-3-kinase/mammalian target of rapamycin kinases (PI3K/mTOR) in the signaling PI3K/mTOR pathways (e.g., SAR245409, AZD8055, temsirolimus, everolimus, or BEZ235); a P13K inhibitor (e.g., SAR2455408 or GDC-0941); an antimetabolite (e.g., fluorouracil, capecitabine, gemcitabine, FOLFIRI); an antimitotic agent (e.g., docetaxel, paclitaxel, pemetrexed); an inhibitor of an epidermal growth factor receptor (EGFR)(e.g., cetuximab); a tyrosine kinase inhibitor (e.g., imatinib, erlotinib, gefitinib); an alkylating agent (e.g., dacarbazine, tomozolomide); or an inhibitor of B-raf protein kinase (BRAF) (e.g., dabrafenib, RO5212054, ARQ 736, or vemurafenib).

MEK inhibitors and their use

MEK inhibitors have been previously described and are being developed as cancer therapeutics either in a monotherapy or as a combination therapy with another class of anti-cancer therapeutic compounds. MEK inhibitors have been described in several publications, including WO2010138377, WO2009153554, WO2009093009, WO2009013462, WO2009093013, WO2008020206, WO2008078086, WO2008120004, WO 2008125820, WO2009093008, WO2009074827, WO2009093009, WO2010108652, WO2010105110, WO2010105082, WO2009129246, WO2009018238, WO2009018233, WO2008089459, US20080255133, US20080058340, WO2008124085, WO2008076415, WO2008021389, WO2010051935, WO2010051933, WO2009129938, WO2009021887, WO2008101840, US20090275606, US20090246198, WO2008055236, WO2010003025, WO2010003022, WO2007096259, WO2008067481, WO2008024724, WO2008024725, and WO20100145197.

Examples of these compounds are here shown:

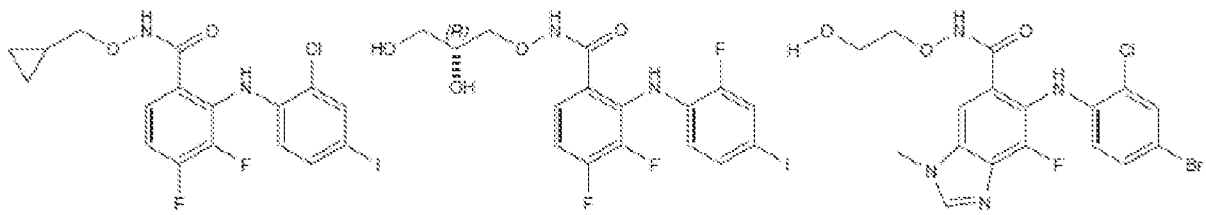

CI-1040/PD-184352
Pfizer

PD-0325901
Pfizer

ARRY-142886/AZD 6244/Selumetinib
Array BioPharma/AstraZeneca

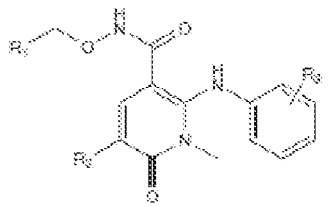

ARRY-424704/AZD8330
Array BioPharma/AstraZeneca

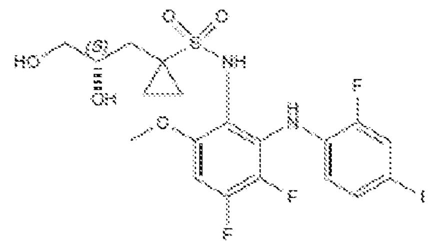

RDEA-119/Bay-869766
Ardea Biosciences/Bayer

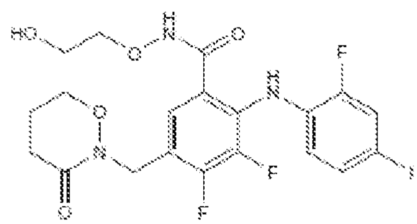

RO4987655/CH4987655
Hoffmann-La Roche

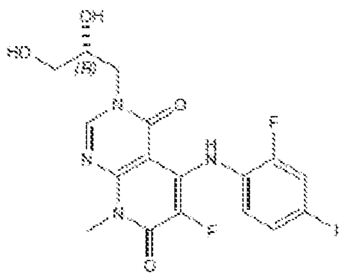

TAK733
Millennium/Takeda

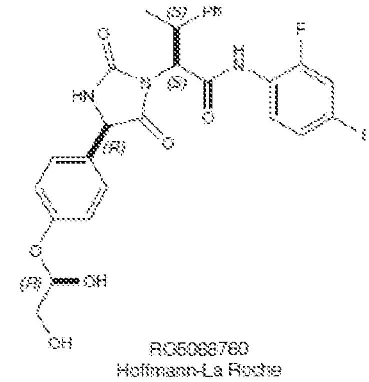

RO5068760
Hoffmann-La Roche

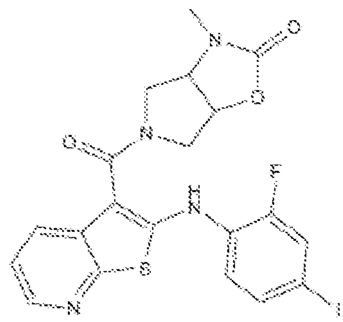

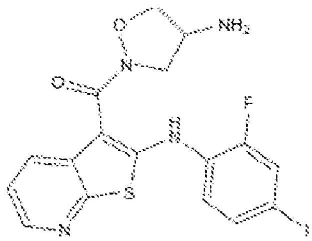

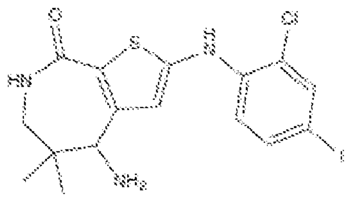

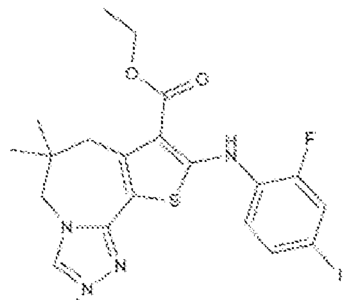

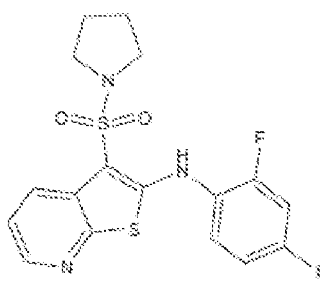

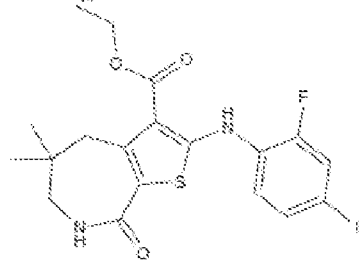

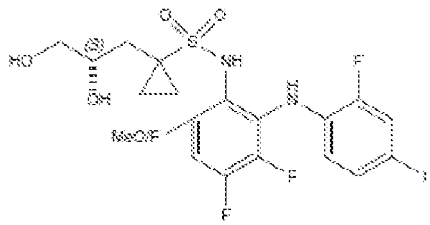

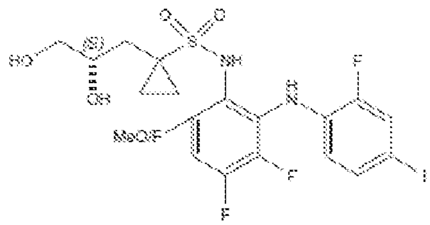

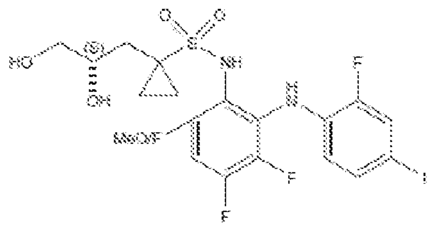

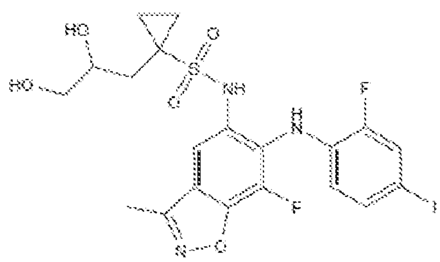

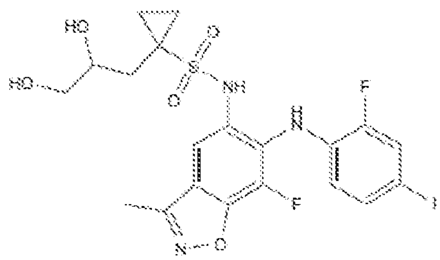

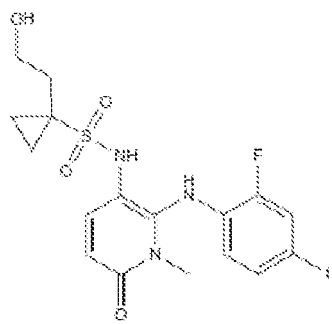

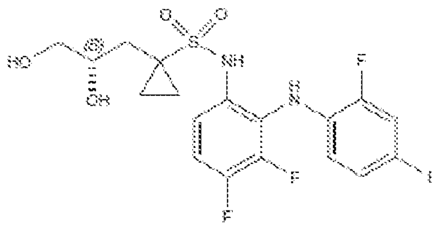

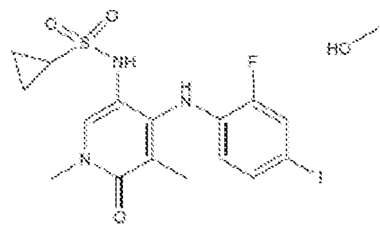

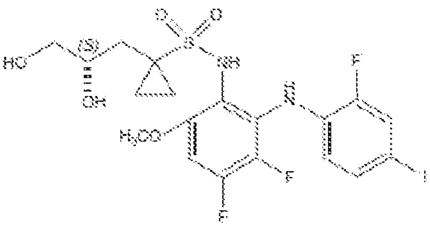

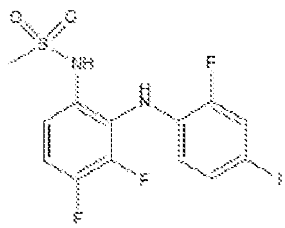

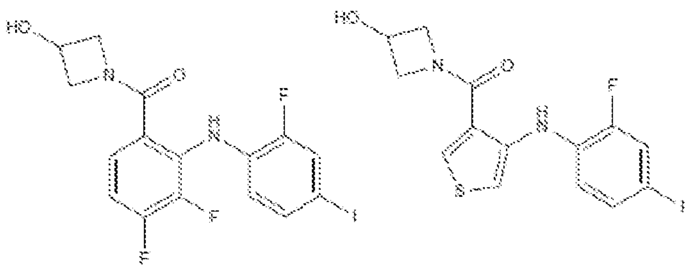

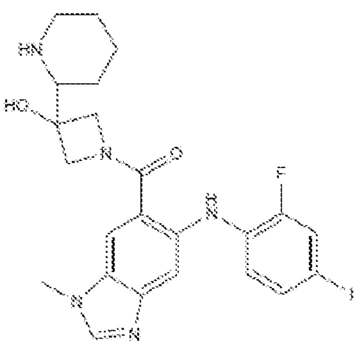

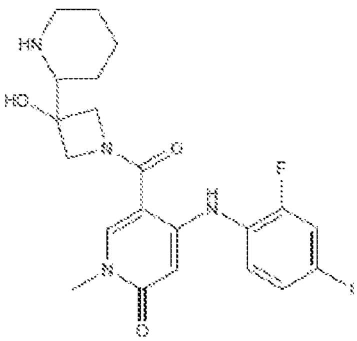

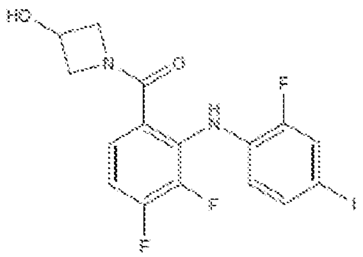

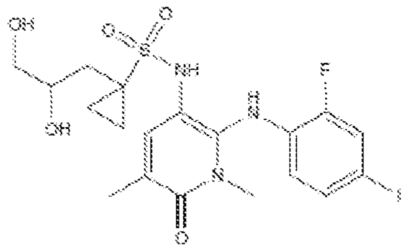

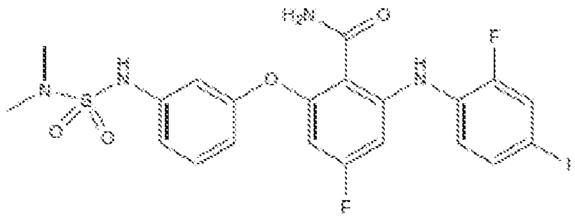

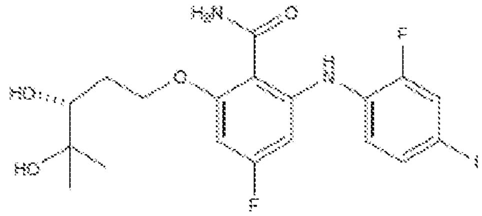

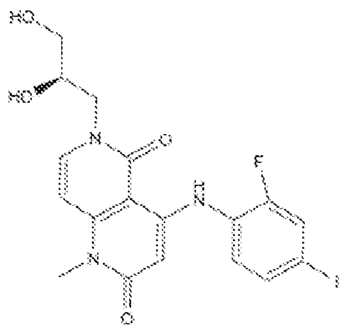

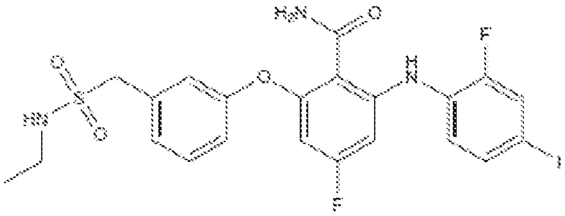

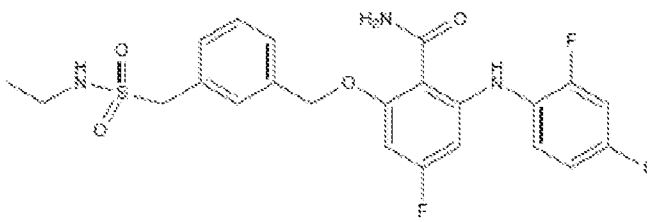

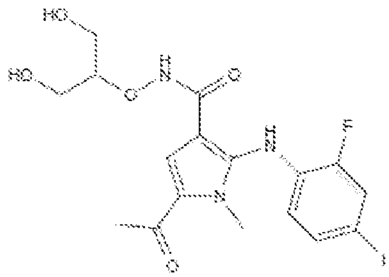

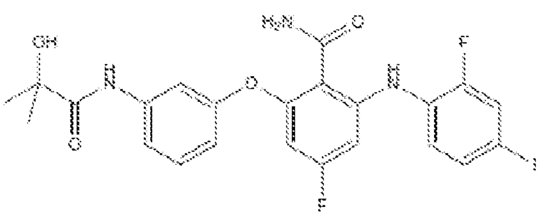

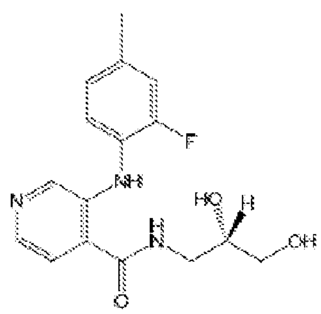

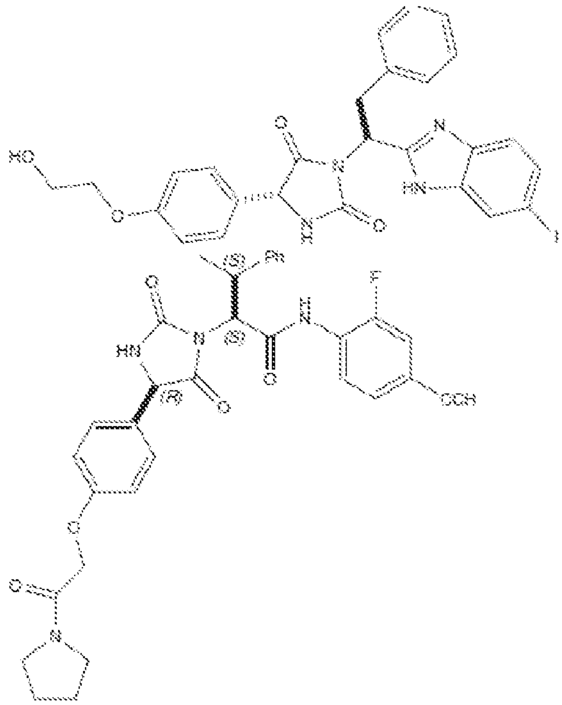

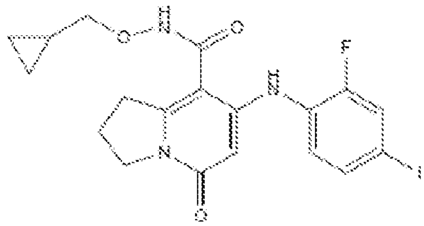

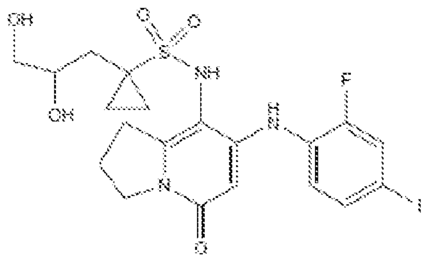

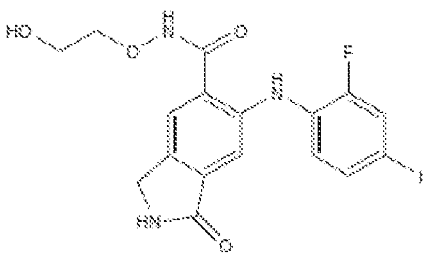

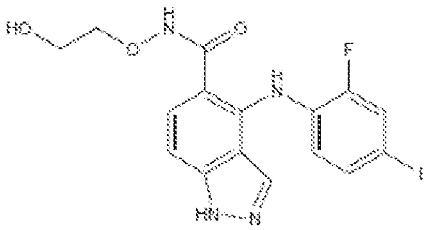

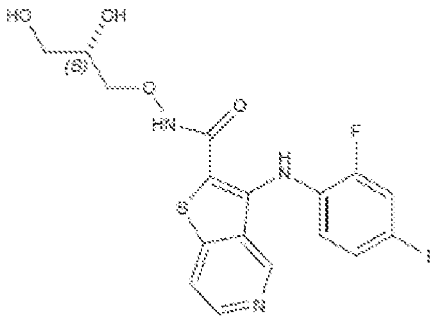

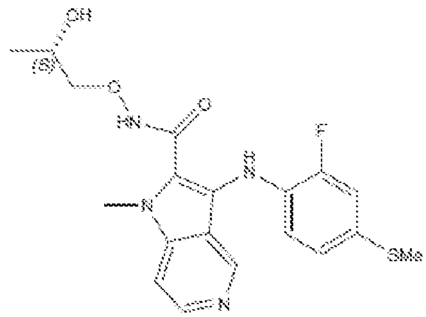

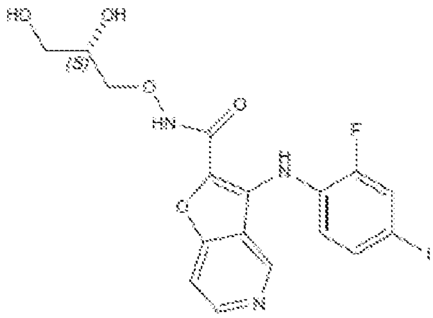

The bioactive MEK inhibitors described above are prepared in pharmaceutical formulations for administration to cancer patients.

Many of the MEK inhibitors currently being developed are oral formulations. For example, Pimasertib is supplied as 15, 30 or 60 mg hard gelatin capsules, taken orally once or twice a day. Treatment is continued until disease progression, intolerable toxicity, or a decision to discontinue treatment.

### Treating adverse events in cancer patients ongoing MEK inhibitor treatment

Severe side effects are reported to occur from administration of MEK inhibitor, including including serious skin rashes, diarrhea, asthenia, and dangerous drops in white blood cell counts, and retinal detachment.

IFNγ can be used to treat these side effects. For treating adverse events other than in the eye, various modes of administration of IFNγ are known to be safe and effective. Aerosolized IFNγ has been shown to be a safe and effective means of administering IFNγ to the lung by inhalation (reviewed by Thipphawong, 2006, Adv Drug Del Rev 58:1089-1105). Commercial nebulizers are suitable for this mode of administration, e.g., the AERx system (Aradigm, Hayward CA). IFNγ is known to provide an inflammatory signal by down regulating epithelial $Na^+$ channel and upregulate $Ca^{2+}$-dependent $Cl^-$ secretion, and to thereby counterbalance the effects of cystic fibrosis and to favor hydration of the airway surface (Galietta et al., 2004, Proc Am Thorac Soc 1:62-65).

Delivery of IFNγ to the liver is known to be effective by means of liposomes comprising amphipathic lipids such as 1,2-distearoyl-sn- glycero-3-phosphocholine, cholesterol, dicetyl phosphate, 1,2-dipalmitoyl-sn-glycerol-[3- phospho-rac-(1-glycero)], 1,2-distearoyl-sn-glycero-3-phosphoethanolamine, 1,2- dipalmitoyl-sn-glycero-3-phosphoethanolamine-N-(succinyl), preferably containing a biotin molecule to assist in targeting (see EP 1883395 A2).

Delivery of IFNγ to the intestine may be effected by oral delivery in a thin film comprising water- soluble polymer such as pullulan, hydroxypropyl cellulose, polyvinyl pyrrolidone, carboxymethyl cellulose, polyvinyl alcohol, sodium alginate, polyethylene glycol, xanthan gum, tragacanth gum, guar gum, acacia gum, Arabic gum, polyacrylic acid, methylmethacrylate copolymer, carboxyvinyl polymer, amylase, high amylase starch, hydroxypropylated high amylase starch, dextrin, pectin, chitin, chitosan, levan, elsinan, collagen, gelatin, zein, gluten, soy protein isolate, whey protein isolate, or casein (see WO 2010002418). In these formulations, IFNγ is preferably located in pH-sensitive microparticles comprising a copolymer of methacrylic acid or acrylic acid, such as a Eudragit- style copolymer; a pluronic polymer; a chitosan, a chitosan derivative or a combination thereof.. The Eudragit- style copolymer may be comprised of Eudragit® L polymer, e.g., Eudragit® L100-55, and Eudragit® S polymer, e.g.,, Eudragit® SIOO. Intestinal delivery of IFNγ by T cells has been shown to be effective in treating intestinal disease caused by rotavirus (Yuan, et al., 2008, Vaccine 26:3322-31).

### Adverse effects of MEK inhibitor in the eye

IFNγ can be used to treat these side effects of MEK inhibitor in the eye. What is described is the means of detecting and measuring visual disturbances, and the means of using IFNγ to treat the effects.

### Measuring Visual Disturbances: NEI/ETDRS Methods for Determining Refraction and BCVA

This standard describes a single method for the measurement of visual acuity (which is strongly influenced by the methods used in the ETDRS and AREDS protocols, below) so that measurements obtained using the procedures listed below can be compared within and between sites as described in Ferris FL, et al.,1982, Am J Ophthalmol, 94:91-96. Three methods are described to select the optical correction that will be used to measure visual acuity. Since determining this optical correction by manifest refraction is usually the lengthiest part of the evaluation of visual acuity, the three procedures listed below are, in the order from the most to the least time-consuming: measurement of best corrected visual acuity (BVCA) with required manifest refraction, measurement of corrected visual acuity with conditional manifest refraction, and measurement of corrected visual acuity without manifest refraction.

#### *1. Measurement of BCVA with required manifest refraction*

This technique is utilized exclusively at visits to compare results obtained where visual acuity is measured in a clinical research protocol. Best-corrected visual acuity is preferably obtained at baseline and on the additional study defined visits.

#### *2. Measurement of corrected visual acuity with conditional manifest refraction*

This technique requires a manifest refraction only if visual acuity declines or improves by 10 or more ETDRS letters (0.20 logMAR) as compared to the relevant baseline measurement. In the event of such a change in the visual acuity score, the baseline measurement is preferably changed to the score that triggered the "event."

#### *3. Measurement of corrected visual acuity without manifest refraction*

This technique is preferably used when visual acuity is not used as a study variable. This standard indicates that visual acuity should be measured only once in each participant visit (i.e., *not* without correction, with a pinhole, and

after manifest refraction), so as to preserve the relative unfamiliarity of Chart 1 and Chart 2. The only exception is when Procedure II results in a 10 letter change in visual acuity (in either eye). In this case, a retest of visual acuity (of both eyes) after a manifest refraction is required.

Visual acuity may not be required to be measured in every clinic visit. For example, if a participant with a disease that is not expected to be subject to day-to-day fluctuations in acuity, who has been examined the previous week, returns to the clinic for an additional diagnostic procedure, e.g., perimetry or angiography, it would be unnecessary to retest visual acuity at the second visit.

Participants' pupils are not dilated at the time of visual acuity testing at any study visit. Pinhole acuity will not be tested. Visual acuity is optionally initially assessed utilizing the participant's current distance glasses or the previously obtained manifest refraction. Participants are asked to read the letters on the standard ETDRS Visual Acuity Chart. They start reading from the top left-most letters-first with the right eye and then with the left eye. A visual acuity score is calculated.

## PROCEDURE 1: MEASUREMENT OF BCVA WITH REQUIRED MANIFEST REFRACTION

The visual acuity of participants is measured using a set of three Lighthouse Distance Visual Acuity Test charts (second edition), which are modified ETDRS Charts 1, 2 and R. If participants are illiterate, alternate versions of the "E" chart are available that also meet the requirements. Use of a retro-illuminated chart box is recommended but optional. For either retro-illuminated or front-lighted charts, the illumination of the charts must be even and meet the standards noted below. The charts and boxes are manufactured by Optelec US (Vista, CA) or Precision Vision (LaSalle, IL).

Visual acuity testing is required at a distance of four meters and, for participants with sufficiently reduced vision, at one meter. The 4-meter distance is preferably marked clearly and permanently and the 1-meter distance is preferably measured, with a 1-meter stick, with the participant in a chair.

Visual acuity charts 1 and 2 are used for testing the right and left eye, respectively, and Chart R is used for refraction. The features of the charts are five high-contrast Sloan letters in each of 14 lines of equal difficulty, and a geometric progression of letter size (and, thus, an arithmetic progression of the logarithm of minimum angle of resolution, logMAR) from line to line. Charts 1, 2 and R have different letter sequences. Participants are preferably prevented from seeing Charts 1 and 2 until refraction has been completed and the visual acuity test begins. If a box is not used to hold the charts, the charts are preferably mounted at a height such that the top of the third row of letters (0.8 logMAR) is 49 $\pm$ 2 inches from the floor.

The dimensions of an optionally used retro-illuminated light box are 24-¾ inches high by 25-¾ inches wide by 7 inches deep. The box can be mounted on a wall or on a cylindrical stand manufactured by Lighthouse Low Vision Products. The stand is mounted on a five-pronged wheel base, with each prong about 14 inches long; two of the five wheels are lockable. The rear of the box provides storage space for the two charts not being used. When the box is mounted on the stand, its height can be varied. The light box is preferably mounted at a height such that the top of the third row of letters (0.8 logMAR) is 49 $\pm$ 2 inches from the floor.

Room illumination is preferably between 50 and 125 foot candles as measured with a photometer held four feet from the floor and directed towards the ceiling. The chart is preferably evenly illuminated either in a retro-illuminated visual acuity light box or mounted on an evenly illuminated perpendicular wall at the specified lighting levels.

A distance of 4.00 meters (13 feet and 1.5 inches, or 157.5 inches) is used between the participant's eyes and the visual acuity chart for the 4-meter test. Preferably, the 4.00 meter measurement is exact. The permitted tolerance is only $\pm$ 2 cm (0.02 meter) from cornea to chart surface in the 4-meter lane. For testing at one meter, the distance is preferably 1.00 $\pm$ 0.01 meters (39 and 3/8 inches). A measuring tape or meter stick is preferably always be available to verify the chart distance, even if the examining chair is immovable or if reference marks are placed on the floor or walls. (Custodial and other staff have been known to move room furnishings about and clean-off marks from the floor or wall while performing their duties, necessitating re-establishing the correct distances for the lane.)

### Refraction Technique

The technique described below is used whenever a manifest refraction and BCVA measurement is indicated by the study protocol. Any standard visual acuity chart, such as Refraction Chart R or a Projecto-Chart, and any test distance are used for determining the best lens correction in each eye, though using the 4-meter lane is recommended. If the standardized test (4-meters, Chart R) is not used, however, an over-refraction with spheres is preferably performed, using Chart R at four meters prior to testing visual acuity. Charts 1 and 2 are not used for refraction, only for visual acuity testing. The right eye is refracted first and then the left eye.

If the participant wears contact lenses and has glasses, he or she is told not to wear the contact lenses on the day of the examination. If the participant appears for the examination wearing contact lenses (because he or she has forgotten to follow the instructions or because he or she has no glasses), the contact lenses are removed and refraction

and visual acuity testing should not begin for at least half an hour. Longer periods for corneal reshaping are needed if the participant is wearing hard contact lenses.

The result of a subjective refraction on a previous visit can be used as the beginning approximate refraction. If this is not available, then the following procedures are followed:

(a) If the participant's uncorrected visual acuity is 20/200 or better and the participant does not have glasses for distance vision, the beginning approximate refraction is no lens correction (plano);
(b) If the participant's uncorrected visual acuity is less than 20/200 in either eye with the participant's present distance glasses (or without correction, if the participant does not have glasses), retinoscopy is preferably performed by an examiner proficient in this procedure. An acceptable alternative is to conduct an arbitrary trial with any lenses to bring acuity to 20/200 or better; another is to use an automated refractor. The lens corrections obtained are used as the beginning approximate refraction for determining best-corrected visual acuity;
(c) If the participant's visual acuity is 20/200 or better with the participant's present distance glasses, the glasses are measured with a lensometer and these measurements are used as the beginning approximate refraction.

The trial frame is placed and adjusted on the participant's face so that the lens cells are parallel to the anterior plane of the orbits and centered in front of the pupils. (It is permissible to use a Phoroptor for subjective refraction. However, for testing visual acuity the lenses from the final Phoroptor refraction must be placed in a trial frame and the final sphere must be rechecked in the 4-meter lane. See information below about refining final spherical power.) The left eye is occluded and the beginning approximate refraction, as determined above, is placed in the right lens cells with the cylindrical correction anterior. Standard eye charts are read at a distance of 10 to 20 feet directly or with a mirror (closer if visual acuity is too poor for the participant to see the largest letters on the chart at this distance). Using the standard 4-meter visual acuity lane will obviate the need to switch locations between refraction measurements and acuity measures, so this is preferred.

**Determination of spherical refraction**

The visual acuity of the right eye is assessed and noted. A +0.50 sphere is then held in front of the right eye and the participant is asked if the vision is "better," "worse," or "no different" while he or she is looking at the smallest line read well.

1. If vision is improved or there is no change, the sphere in the trial frame is replaced with one that is one-half diopter more plus. The +0.50 sphere is held in front of the right eye again and the participant is asked again if the vision is "better," "worse," or "no different." This process of increasing the plus sphere in the trial frame is repeated until the participant says that the +0.50 sphere held in front of the trial frame makes the vision worse. When the participant responds that the vision is made "worse," the lens should be left in place for 10 to 15 seconds in an attempt to evaluate whether the participant is accommodating. If the vision clears during this period, the +0.50 sphere may be added again and succeeding attempts to evaluate additional plus lenses should be accompanied with a 10- to 15-second delay. If there is no evidence of unrelaxed accommodation, the delay period while assessing plus lenses is not necessary at any time further in the examination.
2. Whenever the participant says that the vision is "worse" and remains worse, the +0.50 sphere is removed from in front of the trial frame. By this process, the highest-plus or least-minus sphere that is tolerated without blurring the participant's vision is determined. After determining this highest-plus or least-minus sphere, the participant is asked to read the smallest line possible.
3. Next, a -0.37 sphere is held in front of the trial frame and the participant is asked if the vision is "better," "worse," or "no different." If vision *is* improved, the participant is requested to read the chart and if at least *one* more letter is read, the sphere in the trial frame is replaced by a sphere that is 0.25 diopter less plus. In certain situations, the participant is unable to read more letters, but is convinced that the vision is actually improved. If the examiner believes that this is the case, the additional minus lens can be added. At any stage in the examination, no more than 0.25 diopters of minus should be added without an increase in the number of letters read correctly. The additional minus lens should not be added if the participant reads fewer letters but states that acuity is better. There is a general attempt in this refraction protocol to avoid "over-minusing" the participants. However, when plus cylinders are in the refraction, one must be careful not to unnecessarily withhold minus which may be necessary for the participant to accept the needed plus cylinders later in the refraction. Minus spherical power is added in -0.25-diopter increments until the participant shows no further improvement in vision. If minus power is added, a +0.50 sphere is tried again to determine if more plus will be accepted.
4. If the participant says the vision is "not different" or "worse," no minus power should be added and the spherical determination is complete.

Herein, only plus cylinder techniques are presented. Minus cylinders may be used instead of plus cylinders to determine the best correction for the cylinder power and axis. If minus cylinders are used, the procedures must be revised to reflect the change in sign.

**Cylinder axis determination**

If the beginning approximate refraction contains a cylinder correction, changes in cylindrical axis are tested by adding a 0.25, 0.37, or 0.50 diopters cross-cylinder, first with the positive axis 45° to one side of the cylinder axis, and then with the positive axis 45° to the opposite side of the cylinder axis. Since neither position may produce a clear image, the participant is encouraged to select the position producing "less blur" while fixing on a single round letter on the line above the lowest line on the chart he or she is able to read when the cross-cylinder is not held up before the trial frame. If the participant cannot choose between the two positions of the cross-cylinder at the beginning of this test, the axis of the cylinder is moved 5° to 15°, first in one direction and then in the other, with the cross-cylinder being checked in each position to confirm that the original axis was indeed correct. If the participant prefers one position of the cross-cylinder to the other and the cylinder in the trial frame is plus, the axis of the cylinder is moved 5° to 15° toward the positive axis of the cross-cylinder when it is in the position found to be less blurry by the participant.

When the power of the cylinder is low or the participant's discrimination is poor, larger shifts produce more clear-cut answers. The cross-cylinder is tried again with the positive axis 45° first to one side and then to the opposite side of the new cylinder axis to determine which position is producing less blur.

If the participant finds one position less blurry, the axis of the plus cylinder is moved toward the positive axis of the cross-cylinder. Testing for change of axis is repeated until the participant finds neither position definitely better than the other.

**Cylinder power determination.**

Change in cylinder power is tested by adding the cross-cylinder, first with the positive axis and then with the negative axis coincident with the cylinder axis. For this test, the participant is requested to focus attention on a round letter on the lowest line on the chart he or she is able to read. If the participant prefers the positive axis coincident with the cylinder axis, the power of the correcting plus cylinder is increased by an additional +0.25 diopter. If the participant prefers the negative axis coincident with the cylinder axis, the total power of the correcting plus cylinder is reduced by 0.25 diopter. The process is repeated until the participant finds neither position definitely better than the other. As plus cylinder is added, the examiner should recognize that the spherical equivalent of the refraction is being changed. More minus spheres may be needed as plus cylinders are added. When using plus cylinders for every 0.50 diopter of cylinder power added, the sphere should be changed by -0.25 diopter. If, at any time, the preference with the cross-cylinder indicates that cylinder power should be removed entirely, the 0.25 cylinder should be rotated 90° from its original position. The axis should be refined and the power should be tested again.

If the beginning refraction is a "pure" sphere, the presence of astigmatism is tested by arbitrarily placing a +0.25 cylinder at 180° in the trial frame, after having determined the highest-plus or least-minus sphere producing minimal blurring of vision, as described above. The refraction is then continued by using the cross-cylinder to test for cylinder axis and then cylinder power using the cross-cylinder technique outlined above. If, at any time, the preference with the cross-cylinder indicates that cylinder power should be removed entirely, the 0.25 cylinder should be rotated 90° from its original position and the power should be tested again. At this point, if the participant prefers additional power, it should be added. If, on the other hand, the participant prefers to remove the +0.25, it should be removed and the final refraction is then purely spherical. An example of this procedure follows: For example, with a beginning refraction: -2.50 + 0.25 × 37° and use of the cross-cylinder to check cylinder axis indicates that the participant prefers the 37° axis. If, on using the cross-cylinder to check cylinder power, the participant wants the 0.25 cylinder removed, rotate the cylinder to 127° and test for cylinder power again. If additional power is preferred, add it. If the preference with the cylinder at 127° is to remove the 0.25 cylinder, this should be done and the resulting refraction is -2.50 sphere.

**Refining final spherical power**

When neither the power nor the axis of the cylinder can be improved, the power of the sphere is refined by testing with +0.25 sphere and -0.37 sphere and changing the spherical power. If the sphere is changed at this point, the cylinder should be rechecked. This process is repeated until no further significant lens changes are made.

This refraction protocol can be summarized as follows. First, having eliminated any possible accommodation with plus spheres, the spherical equivalent power is placed on the retina. Then the cylinder power and cylinder axis are assessed. This process of checking sphere, cylinder axis and cylinder power is repeated until there are no changes that result in an increased number of letters being read. Ideally, at the end of the refraction, the sphere is checked and the

participant neither tolerates increased plus nor improves with increased minus spheres. Then the axis is checked and no change in axis is indicated. Finally, the cylindrical power is checked and no change in this is indicted. At this point, the refraction is completed. Sometimes this endpoint cannot be reached because there are an unending number of small corrections at each repetition of the process. When it becomes clear that these small changes are not resulting in an increased number of letters read correctly, the examiner terminates the refraction.

The lens corrections obtained in this way for the right eye are recorded in the study records as the corrections obtained by subjective refraction for the right eye. The entire process is repeated for the left eye, and these lens corrections are also recorded in the study records as the corrections obtained by subjective refraction for the left eye.

**Adjustment for non-standardized test conditions during refraction**

If a test distance other than four meters is used for refraction, the participant should be taken to the site of visual acuity testing in the 4-meter lane. At this site, a final adjustment of the sphere should be made at four meters just before visual acuity testing, using Refraction Chart R with appropriate lighting while not allowing the participant to see Chart 1 or Chart 2. If this refraction differs from the initial refraction, this lens correction is recorded in the study records. Similarly, if a Phoroptor is used for the subjective refraction, a final check on the sphere is performed with a trial frame using the 4-meter refraction lane and Refraction Chart R. A change of spherical power in these circumstances does not require rechecking the cylinder power or axis.

**Refraction for participant with poor visual acuity**

If it is not possible to perform a subjective refraction at 10 to 20 feet because visual acuity is too poor for the participant to see the largest letters on the refraction chart at this distance, the refraction should be attempted at one meter. If the subjective refraction can be performed successfully at 1 meter, a +0.75 sphere should be subtracted from the 1-meter refraction to make the correction appropriate for the 4-meter distance. This correction should be noted in the study records in the space provided for distance subjective refraction. (*Note:* Visual acuity is tested first at the 4-meter distance even if the participant cannot be refracted at this distance. If the number of letters read correctly at four meters is 19 or less, visual acuity must also be tested at 1 meter, in which case the +0.75 sphere should be added to the 4-meter refraction.)

**Determining Best-Corrected Visual Acuity: Testing at 4-meters**

Testing of all eyes begins at four meters. First, the right eye is tested with Chart 1 and then the left eye is tested with Chart 2. Each chart should remain hidden from view until the eye in question is ready for testing.

The distance from the participant's eyes to the visual acuity chart is preferably exactly 4.00 meters (13 feet and 1.5 inches, or 157.5 inches). The participant may stand or sit for the 4-meter visual acuity test. If the participant is seated, his or her back should fit firmly touching the back of the chair. The examiner should ensure that the participant is standing or sitting comfortably, that the head does not move forward or backward during the test and that the participant's eyes remain at the 4-meter distance.

The testing procedure for visual acuity is based on the principle that the objective is to test visual acuity and not intelligence or the ability to concentrate or follow or remember instructions (although all of these factors are involved). The participant should be told that the chart has letters only and no numbers. If the participant forgets this instruction and reads a number, he or she should be reminded that the chart contains no numbers and the examiner should request a letter in lieu of the number. The examiner must record which letters were read correctly or incorrectly, not just how many (see Section 0). A Visual Acuity Worksheet of the Chart 1 and Chart 2 letters is used to record this while the examination is underway.

The participant is preferably asked to read slowly (at a rate not faster than about one letter per second) in order to achieve the best identification of each letter and to not proceed until the participant has given a definite response. It may be useful for the examiner to demonstrate the letter-$\alpha$-second pace by reciting "A, B, C,...". If, at any point, the participant reads quickly, he or she is asked to stop and read slowly. If the participant loses his or her place in reading or the examiner loses his or her place (possibly because the letters are read too quickly), the examiner asks the participant to go back to where the place was lost. Examiners never point to the chart or to specific letters on the chart or read any of the letters during the test.

Each letter is scored as right or wrong. Once a participant has identified a letter with a definite single-letter response and has read the next letter, a correction of the previous letter cannot be accepted. If the participant changes a response aloud (e.g., "That was a 'C,' not an 'O'") before he or she has read aloud the next letter, then the change should be accepted. If the participant changes a response after beginning to read the next letter, the change is not accepted.

When the participant says he or she cannot read a letter, he or she is encouraged to guess. If the participant identifies a letter as one of two or more letters, he or she is asked to choose one letter and, if necessary, to guess even if the next letter has already been read. The examiner may suggest that the participant turn or shake his or her head in any manner if this improves visual acuity. If the participant does this, care must be taken to ensure that the fellow eye remains covered. When it becomes evident that no further meaningful readings can be made, despite urgings to read or guess, the examiner should stop the test for that eye.

**Testing at 1-meter**

Eyes reading 19 or fewer letters correctly at four meters are preferably tested at one meter. If the trial frame is to be removed when changing the test distance from four meters to one meter, the testing chart (Chart 1 or 2) should first be removed from view to prevent the participant from reading the chart with the fellow eye.

Before testing at 1 meter, a +0.75 sphere is added to the 4-meter correction already in the trial frame to compensate for the closer testing distance. The participant may stand or sit for the 4-meter test, but must sit for the 1-meter test. The avoidance of any head movement forward or backward is particularly important during the 1-meter test. The participant should be asked to read only the first six lines at one meter, making 30 letters the maximum score attainable at that distance.

After the test of the right eye is completed, occlude the left eye and replace Chart 1 by Chart 2. The test is repeated for the left eye, starting at four meters. When testing of the left eye is completed, Chart 2 should be removed from view; Chart R may be mounted in preparation for the next participant.

**Scoring best-corrected visual acuity**

The examiner records each letter identified correctly by circling the corresponding letter on a Visual Acuity Worksheet in the study records. Letters read incorrectly and letters for which no guesses are made are not marked on the form. Each letter read correctly is scored as one point. The score for each line (which ranges from zero if no letters are read correctly to five letters read correctly) and the total score for each eye are recorded on the Visual Acuity Worksheet after testing is completed. If testing at one meter is not required, 30 points are automatically scored for the 1-meter test. The total combined scores (i.e., the sum of the 4- and 1-meter scores) for each eye are recorded. The approximate Snellen fraction is determined based on the lowest line read with one or fewer mistakes, and is recorded on the Visual Acuity Worksheet in the study records.

**Light perception and no light perception**

If visual acuity is so poor that the participant cannot read any of the largest letters at one meter (i.e., the number of letters read correctly at one meter is zero), light perception should be tested with an indirect ophthalmoscope in a darkened room. The indirect ophthalmoscope light should be in focus at three feet with the rheostat set at maximum voltage. From a distance of three feet, the beam should be directed in and out of the eye at least four times, and the participant should be asked to respond when he or she sees the light. If the examiner is convinced that the participant perceives the light, vision should be recorded as "light perception"; if not, vision should be recorded as "no light perception."

**PROCEDURE 2: MEASUREMENT OF BCVA WITH CONDITIONAL REFRACTION**

Visual acuity is measured with the correction established by manifest refraction on a previous visit. If visual acuity of one or both eyes has decreased or increased ten or more letters compared to the baseline measurement, a manifest refraction of both eyes should be performed. The procedure described in *Procedure I: Measurement of BCVA with required manifest refraction,* should be followed in this case. Otherwise, a manifest refraction is not required, and the visual acuity measured is recorded in the medical record.

If visual acuity is not a study defined endpoint (i.e., if after this change has occurred, the participant will continue to participate in the study and visual acuity will continue being measured as a study variable, the baseline value should be "reset" to this new value, which will be used for future study visits that require this approach for the measurement of visual acuity.

The measurement of visual acuity with conditional refraction requires the use of two scoring sheets. Both copies of scoring sheets should be maintained in the medical record.

**PROCEDURE 3: MEASUREMENT OF BCVA WITHOUT MANIFEST REFRACTION**

Visual acuity is measured in the standard fashion, using a refractive correction obtained by one of the following

methods. First, it is preferred that the result of a subjective refraction on the previous visit is used. If this is not available, then, if the participant wears distance correction, the spectacle correction is measured with a lensometer, and these measurements are used. If the participant does not wear distance correction, then the participant's refraction is measured objectively with the automated refractor and the measurement obtained is used. If automated refractor measurements cannot be obtained, then the measurement is the measurement obtained without correction.

**PROCEDURE 4: MEASUREMENT BY FUNDUS PHOTOGRAPHY**

Fundus photography (also called fundography) creates a photograph of the interior surface of the eye, including the retina, optic disc, macula, and posterior pole (i.e. the fundus). Fundus photography is used by optometrists, ophthalmologists, and trained medical professionals for monitoring progression of a disease, diagnosis of a disease (combined with retinal angiography), or in screening programs, where the photos can be analysed later. Compared to ophthalmoscopy, fundus photography generally needs a considerably larger instrument, but has the advantage of availing the image to be examined by a specialist at another location and/or time, as well as providing photo documentation for future reference. Modern fundus photographs generally recreate considerably larger areas of the fundus than what can be seen at any one time with handheld ophthalmoscopes. Fundus photography generally needs a considerably larger instrument than ophthalmography, but has the advantage of availing the image to be examined by a specialist at another location and/or time, as well as providing photo documentation for future reference.

**PROCEDURE 5: ELECTRORETINOGRAPHY (ERG) AND DIRECT-COUPLED ERG (DC-ERG)**

Dark-adapted ERG and dc-ERG recordings were performed by modifying a previously published protocol (Samuels et al., 2010, J Neurophysiol 104:391-402). Following overnight dark adaptation, control and *miR-155* KO mice were anaesthetized using ketamine/xylazine. 2.5% phenylephrine HCl, 0.5% tropicamide, and 0.5% proparacaine HCl were used to dilate the pupil and to anaesthetize the cornea. A 2.5% hypromellose demulcent solution was used to keep the eye moist throughout the recording. An Espion E2 ERG recording system with ColorDome Ganzfeld illumination (Diagnosys LLC, Lowell, MA), fitted with a heated mouse table, was used to obtain dark-adapted ERG recordings, at light intensities of 0.0001 - 10 cd/s.m$^2$, and dc-ERG recordings - at a light intensity of 10 cd/m$^2$. Responses were recorded from both eyes using either gold-plated electrodes (ERG) or Ag/AgCl microelectrode (WPI, Inc., Sarasota, FL) attached to a 1.5 mm capillary equilibrated with HBSS (dc-ERG). An Ag/AgCl pellet reference electrode was located in the mouth and the ground electrode was a platinum needle placed in the tail, subcutaneously. Mice were tested at 9 months of age.

The major components of the dc-ERG wave were measured as previously described (Wu, et al., 2004, Mol Vis 10:650-54). DC-ERG data were digitized and stored for offline analysis in Matlab (Mathworks). Baseline drift correction was performed to measure the amplitudes of the components of the dc-ERG. This was accomplished by determining the line of best fit to the initial 250 points, extrapolating and subtracting it from the entire recording. The c-wave and fast oscillation components of the dc-ERG were measured from this initial drift corrected response. Similarly an additional baseline drift correction was performed by determining the line of best fit to the final 250 points permitting the measurement of the light peak and off response. Entire trace represents the averaged dc-ERG waveform corrected for baseline drift by subtracting the best fit line through all the data points. Statistical analyses were performed using the Student's *t*-test, $p < 0.05$ is considered statistically significant.

**Treatment with IFNγ**

Certain aspects of the description relate to IFNγ for use in methods for treating decreases in visual acuity, particularly decreases in visual acuity associated with treating cancer by administrating a MEK inhibitor alone or in a combination therapy, that cause the accumulation of fluid in the retina and/or subretinal space, that involve administering an amount of IFNγ to the eye of a patient effective to decrease the amount of fluid present in the subretinal space of the patient, and to treat retinal detachment caused drug treatments.

Further aspects of the description relate to IFNγ for use in methods for decreasing the amount of fluid present in the subretinal space of a patient. Such methods can be used, for example, to treat patients suffering from diseases and disorders associates with the accumulation of fluid in the retina and/or subretinal space. Accordingly, in certain aspects of such methods, the patient suffers from age-related macular degeneration, chronic macular edema, diabetic retinopathy, glaucoma, uveitis, peripheral vitreoretinopathy, or retinal detachment caused by, for example, retinal injury or surgery.

In preferred aspects, the use of IFNγ in such methods involve administering an amount of IFNγ to the eye of the patient effective to decrease the amount of fluid present in the retina and/or subretinal space of the patient.

In preferred embodiments of IFNγ for use in the methods of the invention, IFNγ is administered to the basolateral side of the retinal pigment epithelium. In another embodiment of IFNy for use in the method, IFNγ is administered to the

basolateral side of the retinal pigment epithelium, preferably via a liquid droplet administered to the anterior surface of the eye. Alternatively, the IFNγ can be administered to the basolateral side of the retinal pigment epithelium by subtenon injection or by subretinal injection. In preferred embodiments of the invention, IFNγ is administered to the anterior surface of the eye.

In certain embodiments of the invention IFNγ is used in a combination or adjuvant therapy with pimasertib The present invention also provides a composition comprising IFNγ in a pharmaceutically acceptable carrier, in the form of an aqueous solution, a gel, or a gel-like formulation. The pharmaceutically acceptable carrier is a physiologically compatible vehicle, which may include, for example, one or more water soluble polyethers such as polyethylene glycol, polyvinyls such as polyvinyl alcohol and povidone, cellulose derivatives such as methylcellulose and hydroxypropyl methylcellulose, petroleum derivatives such as mineral oil and white petrolatum, animal fats such as lanolin, polymers of acrylic acid such as carboxypolymethylene gel, vegetable fats such as peanut oil, polysaccharides such as dextrans, glycosaminoglycans such as sodium hyaluronate or hyaluronic acid, salts such as sodium chloride and potassium chloride, lanolin, or glycine. In preferred embodiments of the invention, the carrier is a saline solution or is a CELLUVISC® solution.

When the composition is in the form of an aqueous solution, it may comprise physiologically safe excipients formulated to an osmolarity between 250-350 mOsm and pH 5-9; preferably 280-300 mOsM and pH 7.0 -7.6. When the pharmaceutical formulation is in the form of a gel or gel-like formulation, it is preferably a hyaluronic acid or hyaluronic acid-containing formulation approved for intraocular use.

The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. The composition optionally comprises an intraocular irrigation solution approved for surgical use.

The concentration of IFNγ in the compositions can vary widely, i.e., from less than about 0.01% to more than about 1 %, and will be determined primarily based upon fluid volumes, viscosities, etc., in accordance with the particular mode of administration used.

The compositions may be sterilized by conventional, well known sterilization techniques, or may be sterile filtered. The resulting aqueous solutions may be packaged for use as is, or lyophilized, the lyophilized preparation being combined with a sterile solution prior to administration.

The compositions can be formulated as polymer matrices, hydrogel matrices, polymer implants, or encapsulated formulations to allow slow or sustained release of the compositions. A particularly preferred formulation is a suspension or solution of the delivery system in a topical ocular formulation, such as eye drops.

The dosage of IFNγ administered in the compositions can range from about 5 ng/ml to about 100 mg/ml. In certain embodiments of the invention, IFNγ is administered at a dose of about 20 ng/ml to about 25 mg/ml, at a dose of about 80 ng/ml to about 6 mg/ml, at a doses of about 300 ng/ml to about 1 mg/ml, or at a dose of about 650 ng/ml.

Compositions containing IFNγ can be administered to the eyes of a patient using any suitable means, but are preferably applied to the anterior surface of the eye using methods familiar to those skilled in the art. For example, in certain embodiments of the invention, the compositions are applied to the eye via liposomes. Further, in other embodiments the compositions are infused into the tear film via a pump-catheter system. Another embodiment of the present invention relates to the compositions contained within a continuous or selective-release device, for example, membranes such as, but not limited to, those employed in the Ocusert™ System (Alza Corp., Palo Alto, Calif.). As an additional embodiment, the compositions are contained within, carried by, or attached to, contact lenses that are placed on the eye. Another embodiment of the present invention involves the compositions contained within a swab or sponge that is applied to the ocular surface. Further embodiments of the present invention involve the compositions contained within a liquid spray that is applied to the ocular surface. Still further embodiments of the present invention involve injection of the compositions directly into the lachrymal tissues or onto the eye surface. In particularly preferred embodiments of the invention, the compositions are applied to the surface of the eye using conventional eye droppers.

In some embodiments of the invention, the compositions of the invention are administered directly into the eye, such as to the retina and/or subretinal space. In certain of such embodiments, the compositions are administered by subretinal injection using means familiar to those skilled in the art. In other embodiments, the compositions are administered by subtenon injection, as described, for example, in U.S. patent number 6,413,245.

The compositions of the present invention can be administered in a single dose or in multiple doses. For example, the compositions can be administered at the time of eye surgery. Alternatively, the compositions of the present invention can be administered over a course of treatment ranging from weeks to years. In certain embodiments of the invention, sustained release formulations such as implants are administered for the long-term treatment of diseases and disorders amenable to such modes of administration. In exemplary sustained release formulations, IFNγ is delivered over a period of 24 to 72 hours. In preferred embodiments of the invention, a single dose of the compositions is administered. In alternative embodiments, multiple doses of the compositions are administered, for example, every 12, 24,

36, or 48 hours.

Within further embodiments of the invention, IFNγ is administered to a patient in combination with other active agents, or therapeutic regimens, including for example, photodynamic therapy (e.g., for wet AMD), laser photocoagulation (e.g., for diabetic retinopathy and wet AMD), and intraocular pressure reducing drugs (e.g., for glaucoma).

The following examples are illustrative of certain embodiments of the invention and should not be considered to limit the scope of the invention.

## EXAMPLES

### Example 1: Cell Culture

The research followed the tenets of the Declaration of Helsinki and the NIH Institutional Review Board. Fetal eyes (gestation, 16-18 weeks) were obtained from Advanced Bioscience Resources (Alameda, CA) and adult eyes were obtained from Analytical Biological Services Inc. (Wilmington, DE). Human fetal RPE (hfRPE) and cells from human fetal choroid (hfCH) were isolated and cultured using MEM-α based modified medium as described previously in Maminishkis, A., et al., Invest Ophthalmol Vis Sci, 2006, 47, 3612-3624,. For immunofluorescence localization and fluid transport experiments, cells were seeded in transwell chambers and maintained for 6 weeks before experiments. (Corning Costar, 0.4 μm pores, polyester membrane). The confluent monolayers were monitored for their morphology, pigmentation, polarity, and physiology, and confluent monolayers exhibiting the same properties of native RPE were used, as described in Voloboueva, L.A., et al. Invest Ophthalmol Vis Sci, 2005, 46, 4302-4310 ; Shi, G., et al., Invest Ophthalmol Vis Sci, 2008; Li, R., et al., Invest Ophthalmol Vis Sci, 2007, 48, 5722-5732 ; and Maminishkis 2006.

### Example 2: Electrophysiology

Equivalent circuit analysis and electrophysiological methods have been previously described in Quinn, R.H., et al., Invest Ophthalmol Vis Sci, 1992, 33, 3513-3527; Joseph D.P., et al., J Physiol, 1991, 435:439-463; and Maminishkis 2006.

Calomel electrodes in series with Ringer's solutions and agar bridges were used to measure the transepithelial potential (TEP), and the intracellular microelectrode signals are referenced to either the apical or basal bath to measure the membrane potentials, VA and VB, where TEP = VB - VA. Conventional microelectrodes are made from borosilicate glass tubing of 0.5 mm inner diameter and 1 mm outer diameter with a filament (Sutter Instrument Co., Novato, CA) and are back-filled with 150 mM KCl, and have resistances of 80-200 MΩ.

The total transepithelial resistance, $R_T$ (or TER) and the ratio of the apical to basolateral membrane resistance ($R_A/R_B$) are obtained by passing 4 μA current pulses (8 μA peak to peak) across the tissue and measuring the resultant changes in TEP, $V_A$, and $V_B$. Current pulses are bipolar, with a period of 3 sec. $R_T$ is the resulting change in TEP divided by 4 μA, and $R_A/R_B$ is the absolute value of the change in $V_A$ divided by the change in $V_B$ ($R_A/R_B = i\Delta V_A/i\Delta V_B$). The current-induced voltage deflections are digitally subtracted from the records for clarity. The control Ringer solution for measurements of TEP and $R_T$ contain 120 mM NaCl, 5 mM KCl, 23 mM $NaHCO_3$, 1 mM $MgCl_2$, 1.8 mM $CaCl_2$, and 5 mM glucose. In the Figures showing the results of the electrophysiology experiments, a black bar indicates a solution change at the manifold outside of the recording chamber. In some cases the response onset is variably delayed because of "dead space" in the fluid delivery system and because of thickness variations in the unstirred layer at the apical membrane.

Confluent monolayers of cultured hfRPE were mounted on a nylon mesh support and clamped into a modified Üssing chamber that allowed the rapid exchange of Ringer's solution (≈10 chamber volumes per minute) and the measurement of fast electrical changes in seconds. The electrical connections to the apical and basal chambers were made with Ringer-agar bridges in series with calomel electrodes. Intracellular potentials were recorded with conventional microelectrodes, back-filled with 150 mM KCl, with resistances of 80 to 200 MΩ. The apical (A) and basolateral (B) membrane potentials ($V_A$ and $V_B$) are calculated as the voltage differences between the intracellular microelectrode and the apical and basal bath electrodes, respectively. The transepithelial potential (TEP =$V_B$ - $V_A$) is the voltage difference between the apical and basal bath electrodes, and is a function of the resistances of the apical and basolateral membranes ($R_A$ and $R_B$, respectively).

The RPE electrical properties can be modeled by the equivalent circuit shown in Fig 1. The apical and basolateral membranes of the RPE are each represented as an equivalent electromotive force (E), $E_A$ or $E_B$, in series with resistance, $R_A$ or $R_B$, respectively. The paracellular pathway is represented as a shunt resistor, $R_S$, which is the parallel combination of the junctional complex resistances between neighboring cells and the resistance caused by the less-than-perfect mechanical seal around the circumference of the tissue. Because of this shunt resistance and the differences between the membrane EMFs, a current, Is, flows around the circuit. The observed membrane potentials $V_A$ and $V_B$ are given by:

$$V_A = E_A - I_S \cdot R_A \qquad (1)$$

$$V_B = E_B + I_S \cdot R_B \qquad (2)$$

The effect of this loop current is to depolarize the apical membrane and hyperpolarize the basolateral membrane. The apical and basolateral membrane voltages are electrically coupled via Rs, so that any voltage change at one membrane will be partially shunted to the opposite membrane. For example, if a solution composition change primarily alters $E_B$, without altering, $R_A$, $R_B$, or $R_S$, then the apical membrane voltage will also change. Most of the resultant change in $V_A$ is a passive consequence of the current shunted from the basolateral membrane.

$$\Delta V_A = [R_A]/[R_A + R_S] \cdot \Delta V_B \qquad (3)$$

Equation 3 represents a simplified case to illustrate the fractional amount of basolateral membrane voltage change that can appear at the apical membrane. For example, if $R_S$ were close to zero then this fraction is close to 1 and $\Delta V_A \approx \Delta V_B$. In contrast, if $R_S$ » $R_A$ then $\Delta V_A \approx 0$. The transepithelial resistance $R_T$ (TER) is expressed in terms of the membrane and shunt resistances as follows:

$$R_T = [(R_A + R_B)R_S]/[R_A + R_B + R_S] \qquad (4)$$

For example, if the basolateral membrane conductance increases through a decrease in $R_B$, then $R_T$ decrease and $R_A/R_B$ increase.

**Example 3: Fluid transport**

Confluent monolayers of hfRPE cultured on transwells were mounted in an electrophysiological chamber and transepithelial water flow (Jv) measurements were made with a capacitance probe technique as described previously in Maminishkis 2006; Shi, G., et al., Invest Ophthalmol Vis Sci, 2008, 49, 4620-4630; Edelman, J.L., et al., Invest Ophthalmol Vis Sci, 1991, 32, 3033-3040; Jiang, C., et al., Science, 1993, 262, 424-427; and Maminishkis, A., et al., Invest Ophthalmol Vis Sci, 2002, 43, 3555-3566. In brief, the RPE is mounted in a water-jacketed Űssing chamber and oriented vertically with the apical and basolateral membranes separately exposed to Ringer's solution held in bathing reservoirs. Stainless steel probes (Accumeasure System 1000; MT Instruments, Latham, NY) are lowered into the apical and basolateral bathing wells to measure the capacitance of the air gap between the probe and fluid meniscus. Fluid transport rate $J_V$ ($\mu l \cdot cm^{-2} \cdot hr^{-1}$) is determined by monitoring the fluid movement-induced changes in the air gap capacitance at the apical and basolateral baths. The probes on both sides of the tissue are backed off from the surface of the Ringer's solution during a bathing solution change.

To check that the solution changes per se did not appreciably alter Jv, a control-to-control Ringer's solution change is performed near the beginning of each experiment and at appropriate intervals during the experiment. The capacitance probes are moved away from the bathing reservoirs and fresh control Ringer's solution perfused into the chamber. The fluid transport apparatus also allows continuous monitoring of TEP and RT, but for technical reasons (e.g., solution perfusion rates, TEP/$R_T$ sampling rates, and electrode stability) the initial changes in TEP and RT can only be compared to those seen in the electrophysiology experiments. Experiments are continued only if JV, TEP, and $R_T$ are not appreciably altered by this control-to-control Ringer's solution change. The water-jacketed Űssing chamber is placed in an incubator to maintain steady-state control over temperature, $pCO_2$, and humidity.

Tissue viability was ascertained by recording transepithelial potential (TEP) and total tissue resistance ($R_T$). It should be noted that the solution composition changes in this chamber were relatively slow (≈ 1-2 chamber volumes per minute) and the data sampling rate was once per minute, more than two orders of magnitude slower than the sampling rate in the electrophysiology chamber (see below). Therefore it was not possible to record fast changes, in seconds or minutes, in $J_V$, TEP, or $R_T$. After addition of IFNγ to the apical or basal baths, steady-state $J_V$, TEP, and $R_T$ were recorded for 20 - 30 minutes. In control experiments, successive additions of IFNγ were made to test for the repeatability and reversibility of responses.

**Example 4: Statistical Analysis**

Data are expressed as mean ± SEM; statistical significance (Student's t test, two-tailed) was accepted at $p < 0.05$.

**Example 5: MEK inhibitor disrupts fluid regulation in RPE cells**

Effects of MEK inhibitor on human fetal retinal pigment epithelium (hfRPE) were measured following acute and chronic addition of inhibitor. Experiments were performed using hfRPE monolayers grown on semipermeable inserts with media access to apical and basal sides of the epithelium. In different sets of experiments, MEK inhibitor was added to apical, basal, or both sides of hfRPE mounted in modified Űssing chambers. In chronic experiments hfRPE were pre-treated with 10 μM MEK inhibitor added to apical and basal baths for 24, 48, or 72 hours. For chronic experiments lasting more than 24 hours, transepithelial resistance (TER) was measured daily. Prior to the transfer of the epithelial monolayer to the Űssing chamber, the TER of whole insert was compared to the TER measured in the Űssing chamber. This procedure helps confirm the tissue integrity following the transfer from the cell culture insert to the Űssing chamber. The TER summary data for chronic experiments contains cumulative recordings of TER obtained from inserts and Űssing chambers.

Acute addition of MEK inhibitor to hfRPE in continuously perfused (with Ringer) Űssing chamber (electrophysiology chamber) was discovered to increase transepithelial potential (TEP) and slightly decreased TER (at the highest concentration - 200 μM) (Figs. 2A, 2B and 2C). Addition of MEK inhibitor to the apical bath produced larger electrical responses, in which response size increases monotonically with MEK inhibitor concentration, indicating a classic dose-dependent response. These results demonstrate that apical addition of MEK inhibitor in acute and chronic experiments alters transepithelial resistance and fluid transport in RPE, and provide insight into the effects of MEK inhibitor on fluid accumulation in the eye during chemotherapy.

Short term acute addition of MEK inhibitor to hfRPE monolayer did not produce toxic effects because the response of hfRPE to ATP remains after addition of MEK inhibitor (Fig. 3). ATP regulates hfRPE cell calcium and fluid absorption. In vitro experiments previously showed that ATP increases cytosolic calcium levels and stimulation of ion-coupled apical to basolateral membrane fluid transport by acting on $P2Y_2$ receptors on the apical membrane of the RPE (Petersen et al., 1997, J Neurosci 17:2324-37), and the increased fluid transport is likely generated by an increase in net Cl and K absorption across the epithelium, the latter by blockade of K recycling at the apical membrane (*Maminishkis 2006*).

Chronic incubation of hfRPE with MEK inhibitor (10 μM) for 48-72 hours significantly changed responses to acute application of MEK inhibitor and ATP (Figs. 4A and 4B). Fig. 4A shows the response to MEK inhibitor and ATP in the absence of pretreatment, and further that DMSO (the solubilizing agent for MEK inhibitor) did not alter MEK inhibitor and ATP responses. Fig. 4B shows that 72 hour incubation with MEK inhibitor (10 μM) significantly reduced responses of the cells to both MEK inhibitor and ATP. In addition to these changes, chronic exposure to MEK inhibitor produced a monotonic decrease of TEP and TER of hfRPE over time at all concentrations.

These decreases in responsiveness to MEK inhibitor and ATP were not caused by significant cell death since the TER was quite high and the tight junctions evidently intact, since TER remains greater than 600 $\Omega \cdot cm^{-2}$ even after 72 hours (Figs. 5A and 5B). These results demonstrate that the chronic effects of MEK inhibitor on transepithelial fluid transport and resistance of RPE increase over time from 24 to 72 hours. The results also show that MEK inhibitor alters the ATP-induced electrical responses in chronic exposure

Figs. 6A, 6B and 6C summarizes TEP and TER data for chronic exposure to MEK inhibitor. Acute addition of MEK inhibitor (1 μM for Fig. 6A, 10 μM for Fig. 6B or 50 μM for Fig. 6C) to hfRPE apical side caused significant decrease in Jv. The decrease in Jv was observed at concentrations of MEK inhibitor as low as 1 μM. These results show that MEK inhibitor added to the apical bath produces a significant decrease in Jv.

**Example 6: IFNγ Increases Fluid Transport Across the RPE**

Fluid transport assays were performed as described in Example 2 to examine whether IFNγ induced changes in fluid transport across hfRPE monolayers. IFNγ did increase fluid transport: IFNγ (5 ng/ml in the basal bath) increased $J_V$ by ~8.6 $\mu l \cdot cm\text{-}2 \cdot hr^{-1}$, reflecting an increase in steady-state fluid absorption from the retinal to the choroidal side of the tissue (Fig. 7). The pretreatment did not affect cell viability as measured by transepithelial potential (TEP) and total tissue resistance ($R_T$). The mean $J_V$ increased from 12.9 ± 1.6 to 20.5 ± 3.1 $\mu l \cdot cm\text{-}2 \cdot hr^{-1}$ (mean ± SEM, $p < 0.01$). Rapid changes in TEP or $R_T$ were not recorded.

**Example 7: IFN-γ reverses the effect of MEK inhibitor on RPE cells**

The inventors showed previously that the basolateral membrane of human RPE contains a receptor coupled to a canonical JAK/STAT pathway that can be activated by IFN-γ. Addition of IFN-γ to the basal side of hfRPE significantly increased fluid transport across RPE. This increase can be blocked by CFTR inhibitors in the basal bath. Addition of IFN-γ to basal bath of hfRPE increased steady state Jv from 7 μl•cm-2•hr$^{-1}$ to 16 μl•cm-2•hr$^{-1}$ (Li et al., 2009, Am J Physiol Cell Physiol 297:C1452-65) (Fig. 8). In an animal model of retinal-reattachment, topical addition of IFN-γ activated the JAK/STAT pathway and remove fluid from the subretinal space. This removal was monitored by high resolution OCT (Bioptigen).

The MEK inhibitor -induced decrease in Jv could be reversed by addition of IFN-γ to the basal bath of hfRPE. Acute apical addition of 100 μM MEK inhibitor to hfRPE monolayers treated chronically (72 hours) with MEK inhibitor (10 μM) produced a dramatic decrease of fluid absorption. A corresponding response *in vivo* would produce accumulation of fluid in subretinal space and eventually cause retinal detachment. This decrease in fluid absorption was almost completely restored by addition of IFN-γ to the bath bathing the hfRPE basolateral membrane.

Chronic incubation with MEK inhibitor (72 hours) completely altered hfRPE response to ATP (Fig. 9). Incubation with MEK inhibitor (10 μM) reversed the direction of fluid transport from absorption to secretion (Figs. 10A and 10B). Steady state rate of secretion Jv was -2 μl•cm-2•hr$^{-1}$, which increased after acute addition of 100 μM MEK inhibitor to the hfRPE apical bath. The ATP response to increased absorption was also completely altered in this tissue. Reversed fluid transport (secretion, negative values for Jv) after treatment with MEK inhibitor significantly increased after pulse addition of 100 μM MEK inhibitor to apical bath of hfRPE (Fig. 10A). After 72 hours incubation with 10 μM MEK inhibitor, the weak absorptive function was completely reversed after acute addition of 100 μM MEK inhibitor to the apical bath (Fig. 10B), and normal fluid absorption was partially restored after addition of IFN-γ to basal bath bathing hfRPE.

Acute addition of MEK inhibitor to apical bath bathing significantly reduced baseline $J_V$ hfRPE (Fig. 11A, 100 μM MEK inhibitor, and Fig. 11B, 1 μM of MEK inhibitor). This effect of apical MEK inhibitor is almost completely rescued by basolateral addition of IFN-γ, which reverses the apical MEK inhibitor-induced reduction in fluid absorption. This reversal shows that IFNγ can be used to reduce adverse events (retinal edema) associated with MEK inhibitor therapeutic use.

**Example 8: IFNγ treatment and measurement of macular thickness in patients with CME secondary to uveitis**

A phase I open label clinical trial was conducted aimed at investigating the safety and tolerability of topically applied IFN gamma in patients with uveitic cystoid macular edema. To test whether cystoid macular edema (CME), secondary to uveitis, is caused by the disequilibrium of the JAK/STAT and mTor signal transduction pathways in the retinal pigment epithelium (RPE), IFNγ was topically applied in human patients. Five participants with CME secondary to uveitis received a topical ocular instillation of IFNγ in a Phase I, non-randomized, prospective, uncontrolled, dose-escalation, single-center study. The study involved a one-time instillation or series of instillations of IFNγ on the cornea and measurement of a response with optical coherence tomography (OCT) over a three hour period.

A 25% decrease in macular thickness is observed at a post-instillation as compared to baseline. In the study, the first two participants received one instillation (each instillation contains 10 μg in 0.05 mL solution) on the cornea at time zero, the next two participants received two instillations, 10 minutes apart, for a total dosage of 20 μg, and the final participant received three instillations, 10 minutes apart, for a total of 30 μg. OCT was obtained at -60 minutes, -30 minutes and just before the instillation(s). Repeat OCTs were taken at +30 minutes, +60 minutes and +120 minutes. All participants returned for a one-week safety visit.

The primary outcome was the change in central macular thickness as measured by OCT in response to interferon IFNγ as compared with baseline. Secondary outcomes included changes in macular volume as measured by OCT, visual acuity, intraocular pressure, intraocular inflammation and ocular surface irritation assessed by fluorescein staining of the cornea and conjunctiva to assess toxicity. For safety measurements, the following were measured: the presence of ocular surface irritation assessed by fluorescein staining of the cornea and conjunctiva to assess toxicity; the number and severity of systemic and ocular toxicities and adverse events; and the proportion of participants with a visual loss of ≥ 15 ETDRS letters.

This trial demonstrated that IFN gamma drops were well tolerated, with none of the 5 patients reporting irritation or other new ocular complaints during the 1-week period of drug use, or beyond, employing the same dosing regimen proposed in our protocol. In addition, no patient developed evidence of ocular surface irritation by slit lamp exam. There were no serious adverse events, and most patients showed slight (albeit clinically insignificant) decrease in the thickness and volume of macular edema by OCT.

Topical IFN gamma was also studied in the treatment of serous retinal detachments in patients with central serous chorioretinopathy. The trial is ongoing, with four patients studied so far, and some clinical success noted to date. Painless conjunctival injection with no change in vision was observed in two of the four patients treated to date, using

the drug concentration and frequency proposed in this protocol. This occurred during the second week of a two-week course of therapy. Cessation of drops resulted in resolution of redness within one day in both patients, with no persistent ocular changes.

A single-arm, non-randomized, prospective, single-center pilot study includes five patients with non-resolving macular detachments associated with MEK-inhibitor therapy receive topical IFN gamma in one eye over a two week period, with the other eye serving as a control. As this condition presents bilaterally; the eye with the larger macular detachment by OCT imaging serves as the treatment eye; should the detachments appear symmetrical, the right eye will receive treatment.

In addition to tracking the macular detachments by OCT, patients will undergo electrophysiological testing at baseline and at week 2 to identify possible changes in retinal function resulting from the MEK inhibitor, as well as in response to IFN gamma therapy. Should the investigator feel that additional ERG or EOG testing would prove helpful at other times during the protocol, and should the patient agree to undergo the tests, those may be added.

The regimen of IFN gamma therapy will involve dosing the treated eye four times daily, with each dose consisting of a four-drop series, each drop spaced by 1 minute, for a total of 16 drops delivered per day. The solution concentration will be 200 ug per ml, yielding a total daily topical dose of 112 micrograms (7 micrograms per drop X 16 drops). Punctal occlusion will be employed to reduce systemic absorption.

Scheduled clinic visits will occur at baseline, and weeks 1, 2, 4, and 8. In addition, one of the investigators will conduct a phone interview with the patient at day 1 and week 6, to inquire about ocular complications.

Baseline testing will consist of: best corrected visual acuity, intraocular pressure, dilation, OCT macular imaging (standard and, when possible, high resolution), fundus exam, and color fundus photography. The patient will then receive the first dosing of topical IFN gamma (four drops spaced over 3 minutes), followed by repeat OCT testing 1 hour later.

Patients will continue the 4 times daily dosing of IFN gamma for 2 weeks, returning at week 1, and then at week 2 for repeat testing, which will consist of vision, intraocular pressure, dilation, OCT macula, and fundus exam. Patients will stop IFN gamma after 2 weeks of therapy, and return at week 4 for repeat testing. If the macular detachment had improved at week 2 and subsequently worsened by week 4, the patient may be restarted on a second 2 week cycle of IFN gamma, at the same dosing regimen. In that case, they will be asked to return for an additional visit at week 6, though this is not strictly required by protocol. The final protocol study visit will occur at week 8.

If at any time during the protocol the patient experiences ocular redness or other ocular concerns, they will be evaluated as soon as possible, and the investigator may choose to terminate the IFN gamma drops. The patient may be restarted on the drops if the ocular condition resolves, at the investigator's and patient's discretion. Follow-up visits to manage ocular complications will be arranged independent of and in addition to the prescribed visit schedule of the protocol.

At the discretion of the investigator and patient, patients may be followed for longer than 8 weeks on this protocol.

All participants undergo a dilated ophthalmic examination the day of the testing. One eye is chosen as the study eye. The participants receive the evaluation around 12:00 p.m. Participants then have a OCT at -60 minutes, -30 minutes and just before the ocular instillation. The ocular instillation occurs no earlier than 1:00 p.m. Repeat OCT recordings are taken at +30, +60 and +120 minutes after instillation. There is a window of ± 15 minutes for each OCT test.

OCT testing is performed with the Cirrus™ high-definition OCT (Carl Zeiss Meditec, Inc.) scanner using a 512x128 scan pattern where a 6x6-mm area on the retina is scanned with 128 horizontal lines, each consisting of 512 A-scans per line (a total of 65,536 sampled points) within a scan time of 2.4 seconds. The scanner automatically focuses on the macula. Data for macular thickness calculations are collected from an array of A-scans distributed across the macular using the macular thickness analysis algorithm. The three pre-instillation measurements are averaged to calculate the baseline macular thickness measurement. Post-instillation OCT macular thickness calculations (+30, +60 and +120 minutes) is compared to the baseline measurement separately. A 25% reduction in central macular thickness is observed in treated patients.

Participants perform punctal pressure occlusion for at least one minute in an attempt to prevent systemic absorption of the ocular instillation(s). The instillation(s) of the IFNγ and subsequent testing takes less than one day. At the end of the day's testing, participants are given a two-day supply of preservative-free artificial tears for application QID in the study eye.

All of the study procedures, with the exception of the administration of the ocular instillation(s) of IFNγ, are typical components of the clinical care required for a participant with uveitis. In this study, examinations are performed at the study visits as indicated in the study flow sheet as shown in Table II:

1. Medical/Ophthalmic History
2. Vital Signs
3. Concomitant Medication Assessment
4. Adverse Event Assessment
5. Manifest Refraction using ETDRS methods

6. Slit Lamp Examination
7. Intraocular Pressure (IOP)
8. Dilated Fundus Examination
9. Fluorescein Angiogram (FA)
10. Fundus Autofluorescence (FAF)
11. Optical Coherence Tomography (OCT)
12. Subjective Pain Assessment
13. Hepatitis Screening
14. HIV Testing
15. Chemistry 20 Panel
16. Liver Function Tests
17. Urinalysis (UA), including microscopic
18. Urine Pregnancy Test for Women of Child-Bearing Potential

**Formulation, Dosage, and Storage**

Interferon γ-1b, (Actimmune®, InterMune, Inc, Brisbane, CA 94005), a biologic response modifier, is a single chain polypeptide containing 140 amino acids. Actimmune® is a highly purified sterile solution consisting of non-covalent dimmers of two identical 16,465 Da monomers. Actimmune® is a sterile, clear, colorless solution. Each 50 microliters of solution contains 10 mcg (200,000 IU) of IFNγ with 2 mg mannitol, 36 mcg of sodium succinate and 5 mcg of polysorbate 20 in sterile water for injection. This solution has a pH of approximately 5.2 and an osmolality of 221 mmol/kg.

Actimmune® is commercially available in a single-use vial at a concentration of 100 mcg per 0.5 mL (500 microliters). Vials of Actimmune® are placed in a 2-8° C (36-46° F) refrigerator immediately upon receipt to ensure optimal retention of physical and biochemical integrity. The vials are not frozen, excessive or vigorous agitation will be avoided and the vials will not be shaken. Unentered vials of Actimmune® should not be left at room temperature for a total time exceeding 12 hours prior to use. Vials exceeding this time should be discarded. Commercially available Actimmune® is used in this study. When ordered by the investigator, the exact dose is prepared by drawing the appropriate dose (0.05 mL, 0.10 mL or 0.15 mL) of the commercial solution into a tuberculin syringe. The syringe is capped with a sterile cap and sent to the floor for administration. For each instillation, a second back-up syringe is prepared and dispensed to ensure proper instillation.

**Administration**

Interferon γ-1b is administered as follows:

1. Topical tetracaine 1% or proparacaine 0.5% drops are applied to the study eye surface one to two minutes prior to instillation.
2. The participant applies punctal pressure to his/her non-study eye.
3. The investigator holds the participant's study eye open.
4. A volume of 0.05 mL of IFNγ is instilled on the center of the study eye's cornea from a tuberculin syringe.
5. The participant continues applying punctal pressure to his/her non-study eye for at least one minute post-instillation.
6. Steps 1-5 are optionally repeated ten minutes post-instillation until the appropriate dosage has been administered, if an additional instillation is required.
7. The participant is given a two-day supply of preservative-free artificial tears for application QID in the study eye.

Study investigators will obtain informed consent. The Principal Investigator and the NEI Adverse Event Review Committee monitors data and safety. The EMMES Corporation (EMMES) is assigned as the coordinating center for this trial to conduct data collection, protocol monitoring, data analysis and reporting.

**Alternative Therapies**

Alternatives to participation include continuation with the current standard-of-care, which includes the use of systemic steroids, periocular steroids and systemic immunosuppressive agents, all of which are associated with significant side effects and complications if the treatment is extended and increased.

**Table II: Study Flow Sheet**

| Visit Shedule | Baseline[1] | Study Treatment Visit | | | | | | | | Safety Visit [10] |
|---|---|---|---|---|---|---|---|---|---|---|
| **Time (minutes)** | | **Pre-OCT** | **-60 min** | **-30 min** | **1:00 p.m.[2]** | **+30 min[3]** | **60 min[3]** | **+120 min[3]** | **Post OCT** | |
| Visit Number | 000 | 007 | | | | | | | | 014 |
| **Treatment** | | | | | | | | | | |
| IFNγ | | | | | X[4] | | | | | |
| **General Assessments** | | | | | | | | | | |
| Medical/Ophthalmic History | X | | | | | | | | | |
| Vital Signs | X | X | | | | | | | X | X |
| Concomitant Medications Assessment | X | X | | | | | | | | X |
| Adverse Event Assessment | | X | | | | | | | X | X |
| **Ophthalmie Assessments** | | | | | | | | | | |
| Manifest Refraction | X | | | | | | | | X | X |
| Slit Lamp Examination | X | | | | | | | | X | X |
| Intraocular Pressure (IOP) | X | | | | | | | | X | X |
| Dilated Fundus Examination | X | | | | | | | | X | X |
| Fluorescein Angiogram (FA) | X | | | | | | | | X | |
| Fundus Autofluorescence (FAF) | | X | | | | | | | X | X |
| Optical Coherence Tomography (OCT) | X | | X[5] | X[5] | X[6] | X[5] | X[5] | X[5] | | X |
| Subjective Pain Assessment | | X | | | X[7] | | | | X | X |
| **Laboratory Testing** | | | | | | | | | | |
| Hepatitis Screening | X | | | | | | | | | |
| HIV Testing[8] | X | | | | | | | | | |
| Chemistry 20 Panel | X | | | | | | | | | |
| Liver Function Testing | X | | | | | | | | | |
| Urinalysis (UA), including microscopic | X | | | | | | | | | |

(continued)

| | | | | | | | | | | |
|---|---|---|---|---|---|---|---|---|---|---|
| **Laboratory Testing** | | | | | | | | | | |
| Pregnancy Testing (urine) [9] | | X | | | | | | | | |

[1] Baseline procedures ere completed 1 to 7 days prior to the Study Treatment Visit.
[2] Ocular instillation(s) occurred around 1:00 p.m., but no earlier.
[3] The target time for these procedures was calculated from the last instillation (if the participant receives more than one).
[4] The first two participants received a single instillation (dosage of 10 μg) on the cornea at time zero. The next two participants received two instillations on the cornea administered 10 minutes apart (a total dosage of 20 μg). The final participant received three instillations, each administered 10 minutes apart (a total dosage of 30 μg).
[5] These OCT procedures occurred within ±15 minutes of the target time.
[6] The OCT procedure occurred immediately prior to the first ocular instillation.
[7] The assessment was completed immediately following the last instillation.
[8] The Clinical Center HIV Testing Policy was followed when a positive HIV test result was uncovered.
[9] This test was for women of child-bearing potential and they had a negative test within 24 hours prior to the study medication administration.
[10] The safety visit was completed one week after the Study Treatment Visit with a visit window of ± 7 days

The various features and processes described above may be used independently of one another, or may be combined in various ways. All possible combinations and subcombinations are intended to fall within the scope of this disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed example embodiments. The example systems and components described herein may be configured differently than described. For example, elements may be added to, removed from, or rearranged compared to the disclosed example embodiments.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements, and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

While certain example embodiments have been described, these embodiments have been presented by way of example only, and are not intended to limit the scope of the inventions disclosed herein. Thus, nothing in the foregoing description is intended to imply that any particular feature, characteristic, step, module, or block is necessary or indispensable. Indeed, the novel methods and systems described herein may be embodied in a variety of other forms; furthermore, various omissions, substitutions and changes in the form of the methods and systems described herein may be made without departing from the spirit of the inventions disclosed herein. The accompanying claims and their equivalents are intended to cover such forms or modifications as would fall within the scope and spirit of certain of the inventions disclosed herein.

## Claims

1. A pharmaceutical composition comprising IFNγ for use in a method for treating an increased amount of fluid in the retina or subretinal space caused by administration of a MAP kinase/ERK kinase (MEK) inhibitor to a cancer patient.

2. The pharmaceutical composition comprising IFNγ for the use as claimed in claim 1, wherein the MEK inhibitor is selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766

3. The pharmaceutical composition comprising IFNγ for the use as claimed in claim 1, wherein the MEK inhibitor is pimasertib.

4. The pharmaceutical composition comprising IFNγ for the use as claimed in claim 1, wherein the increased amount of fluid causes retinal detachment, optionally wherein the pharmaceutical composition comprising IFNγ is administered in an amount effective to treat the retinal detachment.

5. The pharmaceutical composition comprising IFNγ for the use as claimed in claim 1, wherein the increased amount of fluid is caused by the effect of the MEK inhibitor on fluid transport across the retinal pigmented epithelium.

6. The pharmaceutical composition comprising IFNγ for the use as claimed in claim 1, wherein the increased amount of fluid causes a visual disturbance, optionally wherein the cancer patient has a solid tumor or hematological malignancy.

7. The pharmaceutical composition comprising IFNγ for the use as claimed in claim 1, wherein the pharmaceutical composition comprising IFNγ is administered in an amount to decrease the fluid present in the retina or subretinal space of the cancer patient.

**8.** The pharmaceutical composition comprising IFNγ for the use as claimed in claim 1, wherein;

a) the pharmaceutical composition comprising IFNγ is administered to the basolateral side of the retinal pigment epithelium, or
b) the pharmaceutical composition comprising IFNγ is administered to the anterior surface of the eye, optionally wherein the pharmaceutical composition comprising IFNγ is administered in a liquid droplet.

**9.** A composition comprising IFNγ for use in a method of treating a cancer patient, wherein the method comprises administering a MAP kinase/ERK kinase (MEK) inhibitor and said composition.

**10.** The composition comprising IFNγ for the use as claimed in claim 9, wherein the MEK inhibitor is selected from the group consisting of pimasertib, selumetinib, GSK1120212, GDC0973, GDC0941, GDC0973/XL518, CI1040/PD184352, PD035901, ARRY438162, RDEA436, TAK733, R05126766, and RDEA119/BAY869766, optionally wherein the MEK inhibitor is pimasertib.

**11.** The composition comprising IFNγ for the use as claimed in claim 9, wherein the MEK inhibitor induces an increase the amount of fluid present in the retina or subretinal space of the cancer patient.

**12.** The composition comprising IFNγ for the use as claimed in claim 11, wherein the increase in the amount of fluid causes a retinal detachment.

**13.** The composition comprising IFNγ for the use as claimed in claim 11, wherein the increase in the amount of fluid causes a visual disturbance.

**14.** The composition comprising IFNγ for the use as claimed in claim 11, wherein the pharmaceutical composition comprising IFNγ is administered in an amount to decrease the fluid present in the retina or subretinal space of the cancer patient.

**15.** The composition comprising IFNγ for the use as claimed in claim 13, wherein the pharmaceutical composition comprising IFNγ is administered in an amount effective to treat the visual disturbance.

**16.** The composition comprising IFNγ for the use as claimed in claim 10, wherein:

a) the pharmaceutical composition comprising IFNγ is administered to the basolateral side of the retinal pigment epithelium, or
b) the IFNγ is administered to the anterior surface of the eye, optionally wherein the pharmaceutical composition comprising IFNγ is administered in a liquid droplet.

**17.** The composition comprising IFNγ for the use as claimed in any one of claims 9-16, wherein the cancer patient has a solid tumor or hematological malignancy.

**18.** IFNγ for use in a method for treating an increased amount of fluid in the retina or subretinal space caused by administration of a MAP kinase/ERK kinase (MEK) inhibitor to a cancer patient.

**Patentansprüche**

**1.** Eine IFNγ umfassende pharmazeutische Zusammensetzung zur Verwendung in einem Verfahren zur Behandlung einer erhöhten Flüssigkeitsmenge in der Netzhaut oder im subretinalen Raum, die durch Verabreichung eines MAP-Kinase/ERK-Kinase (MEK)-Hemmers an einen Krebspatienten verursacht wurde.

**2.** Die IFNγ umfassende pharmazeutische Zusammensetzung zur Verwendung nach Anspruch 1, wobei der MEK-Hemmer aus der aus Pimasertib, Selumetinib, GSK1120212, GDC0973, GDC0941, GDC0973/XL518, CI1040/PD184352, PD035901, ARRY438162, RDEA436, TAK733, R05126766 und RDEA119/BAY869766 bestehenden Gruppe ausgewählt ist.

**3.** Die IFNγ umfassende pharmazeutische Zusammensetzung zur Verwendung nach Anspruch 1, wobei der MEK-Hemmer Pimasertib ist.

**4.** Die IFNγ umfassende pharmazeutische Zusammensetzung zur Verwendung nach Anspruch 1, wobei die erhöhte Flüssigkeitsmenge Netzhautablösung verursacht, wobei gegebenenfalls die IFNγ umfassende pharmazeutische Zusammensetzung in einer zur Behandlung der Netzhautablösung wirksamen Menge verabreicht wird.

**5.** Die IFNγ umfassende pharmazeutische Zusammensetzung zur Verwendung nach Anspruch 1, wobei die erhöhte Flüssigkeitsmenge durch die Wirkung des MEK-Hemmers auf den Flüssigkeitstransport durch das retinale Pigmentepithel verursacht wird.

**6.** Die IFNγ umfassende pharmazeutische Zusammensetzung zur Verwendung nach Anspruch 1, wobei die erhöhte Flüssigkeitsmenge eine Sehstörung verursacht, wobei gegebenenfalls der Krebspatient einen soliden Tumor oder eine maligne hämatologische Erkrankung hat.

**7.** Die IFNγ umfassende pharmazeutische Zusammensetzung zur Verwendung nach Anspruch 1, wobei die IFNγ umfassende pharmazeutische Zusammensetzung in einer Menge zur Verringerung der in der Netzhaut oder im subretinalen Raum des Krebspatienten vorhandenen Flüssigkeit verabreicht wird.

**8.** Die IFNγ umfassende pharmazeutische Zusammensetzung zur Verwendung nach Anspruch 1, wobei:

a) die IFNγ umfassende pharmazeutische Zusammensetzung an die basolaterale Seite des retinalen Pigmentepithels verabreicht wird, oder
b) die IFNγ umfassende pharmazeutische Zusammensetzung auf die Vorderseite des Auges verabreicht wird, wobei gegebenenfalls die IFNγ umfassende pharmazeutische Zusammensetzung in einem Flüssigkeitströpfchen verabreicht wird.

**9.** Eine IFNγ umfassende Zusammensetzung zur Verwendung in einem Verfahren zur Behandlung eines Krebspatienten, wobei das Verfahren die Verabreichung eines MAP-Kinase/ERK-Kinase (MEK)-Hemmers und der Zusammensetzung umfasst.

**10.** Die IFNγ umfassende Zusammensetzung zur Verwendung nach Anspruch 9, wobei der MEK-Hemmer aus der aus Pimasertib, Selumetinib, GSK1120212, GDC0973, GDC0941, GDC0973/XL518, CI1040/PD184352, PD035901, ARRY438162, RDEA436, TAK733, R05126766 und RDEA119/BAY869766 bestehenden Gruppe ausgewählt ist, wobei gegebenenfalls der MEK-Hemmer Pimasertib ist.

**11.** Die IFNγ umfassende Zusammensetzung zur Verwendung nach Anspruch 9, wobei der MEK-Hemmer eine Zunahme der in der Netzhaut oder im subretinalen Raum des Krebspatienten vorhandenen Flüssigkeitsmenge auslöst.

**12.** Die IFNγ umfassende Zusammensetzung zur Verwendung nach Anspruch 11, wobei die Zunahme der Flüssigkeitsmenge eine Netzhautlösung verursacht.

**13.** Die IFNγ umfassende Zusammensetzung zur Verwendung nach Anspruch 11, wobei die Zunahme der Flüssigkeitsmenge eine Sehstörung verursacht.

**14.** Die IFNγ umfassende Zusammensetzung zur Verwendung nach Anspruch 11, wobei die IFNγ umfassende pharmazeutische Zusammensetzung in einer Menge zur Verringerung der in der Netzhaut oder im subretinalen Raum des Krebspatienten vorhandenen Flüssigkeitsmenge verabreicht wird.

**15.** Die IFNγ umfassende Zusammensetzung zur Verwendung nach Anspruch 13, wobei die IFNγ umfassende pharmazeutische Zusammensetzung in einer zur Behandlung der Sehstörung wirksamen Menge verabreicht wird.

**16.** Die IFNγ umfassende Zusammensetzung zur Verwendung nach Anspruch 10, wobei:

a) die IFNγ umfassende pharmazeutische Zusammensetzung an die basolaterale Seite des retinalen Pigmentepithels verabreicht wird, oder
b) der IFNγ auf die Vorderseite des Auges verabreicht wird, wobei gegebenenfalls die IFNγ umfassende pharmazeutische Zusammensetzung in einem Flüssigkeitströpfchen verabreicht wird.

**17.** Die IFNγ umfassende Zusammensetzung zur Verwendung nach einem der Ansprüche 9-16, wobei der Krebspatient einen soliden Tumor oder eine maligne hämatologische Erkrankung hat.

**18.** IFNγ zur Verwendung in einem Verfahren zur Behandlung einer erhöhten Flüssigkeitsmenge in der Netzhaut oder im subretinalen Raum, die durch Verabreichung eines MAP-Kinase/ERK-Kinase (MEK)-Hemmers an einen Krebspatienten verursacht wurde.

## Revendications

**1.** Composition pharmaceutique comprenant IFNγ pour utilisation dans un procédé de traitement d'une quantité accrue de fluide dans la rétine ou l'espace sous-rétinien causée par l'administration d'un inhibiteur de MAP kinase/ERK kinase (MEK) à un patient cancéreux.

**2.** Composition pharmaceutique comprenant IFNγ pour l'utilisation selon la revendication 1, dans laquelle l'inhibiteur de MEK est choisi dans le groupe constitué des pimasertib, sélumétinib, GSK1120212, GDC0973, GDC0941, GDC0973/XL518, CI1040/PD184352, PD035901, ARRY438162, RDEA436, TAK733, R05126766 et RDEA119/BAY869766.

**3.** Composition pharmaceutique comprenant IFNγ pour l'utilisation selon la revendication 1, dans laquelle l'inhibiteur de MEK est le pimasertib.

**4.** Composition pharmaceutique comprenant IFNγ pour l'utilisation selon la revendication 1, la quantité accrue de fluide causant un décollement de la rétine, la composition pharmaceutique comprenant IFNγ étant facultativement administré en une quantité efficace pour traiter le décollement de la rétine.

**5.** Composition pharmaceutique comprenant IFNγ pour l'utilisation selon la revendication 1, la quantité accrue de fluide étant causée par l'effet de l'inhibiteur de MEK sur le transport de fluide à travers l'épithélium pigmentaire rétinien.

**6.** Composition pharmaceutique comprenant IFNγ pour l'utilisation selon la revendication 1, la quantité accrue de fluide causant un trouble de la vision, le patient cancéreux ayant facultativement une tumeur solide et/ou une malignité hématologique.

**7.** Composition pharmaceutique comprenant IFNγ pour l'utilisation selon la revendication 1, la composition pharmaceutique comprenant IFNγ étant administrée en une quantité pour diminuer le fluide présent dans la rétine ou l'espace sous-rétinien du patient cancéreux.

**8.** Composition pharmaceutique comprenant IFNγ pour l'utilisation selon la revendication 1, où ;

a) la composition pharmaceutique comprenant IFNγ est administrée au côté basolatéral de l'épithélium pigmentaire rétinien, ou
b) la composition pharmaceutique comprenant IFNγ est administrée à la surface antérieure de l'oeil, la composition pharmaceutique comprenant IFNγ étant facultativement administrée dans une gouttelette de liquide.

**9.** Composition comprenant IFNγ pour utilisation dans un procédé de traitement d'un patient cancéreux, le procédé comprenant l'administration d'un inhibiteur de MAP kinase/ERK kinase (MEK) et de ladite composition.

**10.** Composition comprenant IFNγ pour l'utilisation selon la revendication 9, dans laquelle l'inhibiteur de MEK est choisi dans le groupe constitué des pimasertib, sélumétinib, GSK1120212, GDC0973, GDC0941, GDC0973/XL518, CI1040/PD184352, PD035901, ARRY438162, RDEA436, TAK733, R05126766 et RDEA119/BAY869766, l'inhibiteur de MEK étant facultativement le pimasertib.

**11.** Composition comprenant IFNγ pour l'utilisation selon la revendication 9, dans laquelle l'inhibiteur de MEK induit une augmentation de la quantité de fluide présent dans la rétine ou l'espace sous-rétinien du patient cancéreux.

**12.** Composition comprenant IFNγ pour l'utilisation selon la revendication 11, l'augmentation de la quantité de fluide causant un décollement de la rétine.

**13.** Composition comprenant IFNγ pour l'utilisation selon la revendication 11, l'augmentation de la quantité de fluide causant un trouble de la vision.

**14.** Composition comprenant IFNγ pour l'utilisation selon la revendication 11, la composition pharmaceutique comprenant IFNγ étant administrée en une quantité adéquate pour diminuer la quantité de fluide présent dans la rétine ou l'espace sous-rétinien du patient cancéreux.

**15.** Composition comprenant IFNγ pour l'utilisation selon la revendication 13, la composition pharmaceutique comprenant IFNγ étant administré en une quantité efficace pour traiter le trouble de la vision.

**16.** Composition comprenant IFNγ pour l'utilisation selon la revendication 10, où :

a) la composition pharmaceutique comprenant IFNγ est administré au côté basolatéral de l'épithélium pigmentaire rétinien, ou
b) la composition pharmaceutique comprenant IFNγ est administrée à la surface antérieure de l'oeil, la composition pharmaceutique comprenant IFNγ étant facultativement administrée dans une gouttelette de liquide.

**17.** Composition comprenant IFNγ pour l'utilisation selon l'une quelconque des revendications 9 à 16, le patient cancéreux ayant une tumeur solide ou une malignité hématologique.

**18.** IFNγ pour utilisation dans un procédé pour traiter une quantité accrue de fluide dans la rétine ou l'espace sous-rétinien causée par l'administration d'un inhibiteur de MAP kinase/ERK kinase (MEK) à un patient cancéreux.

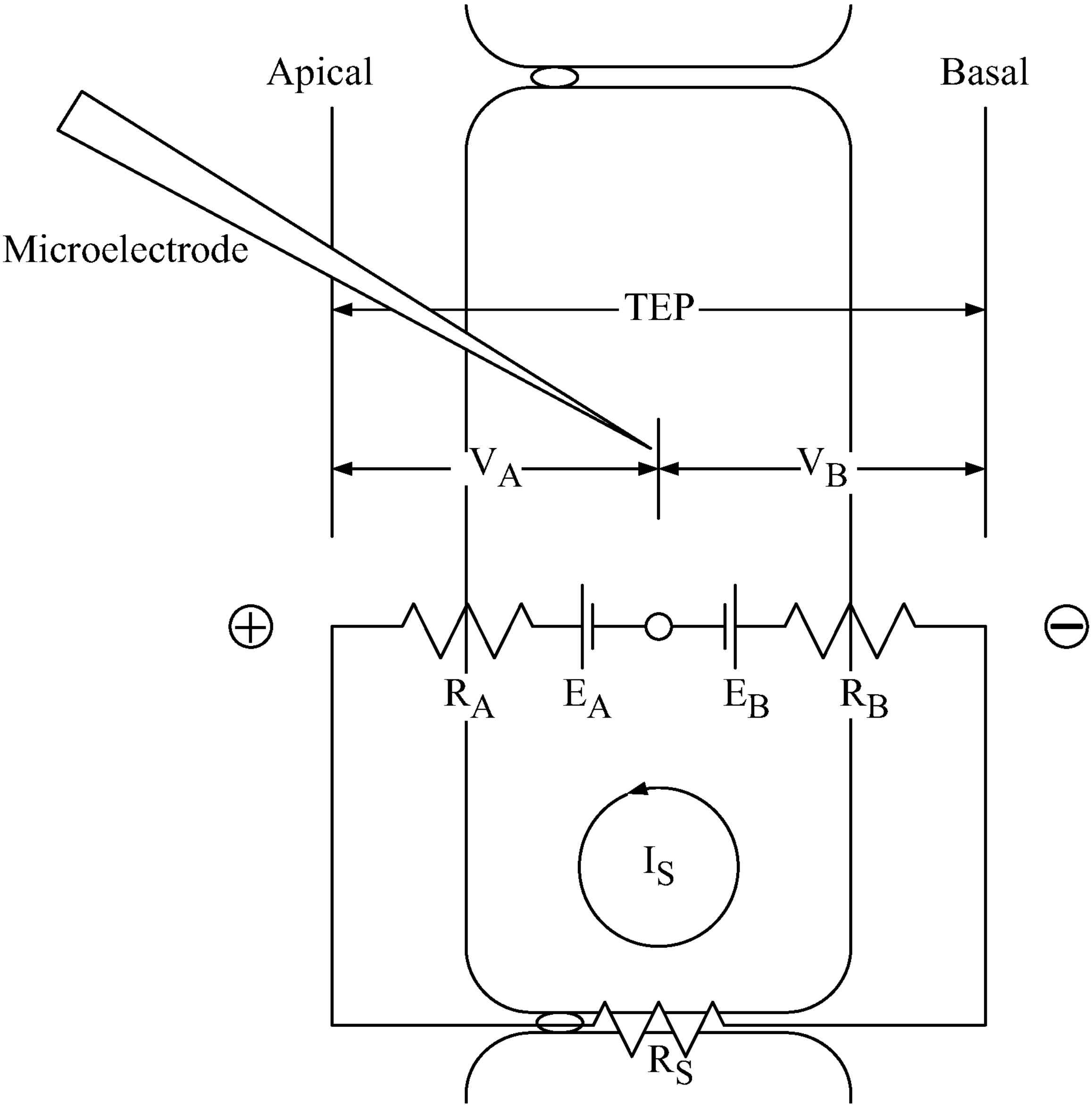


FIG. 1

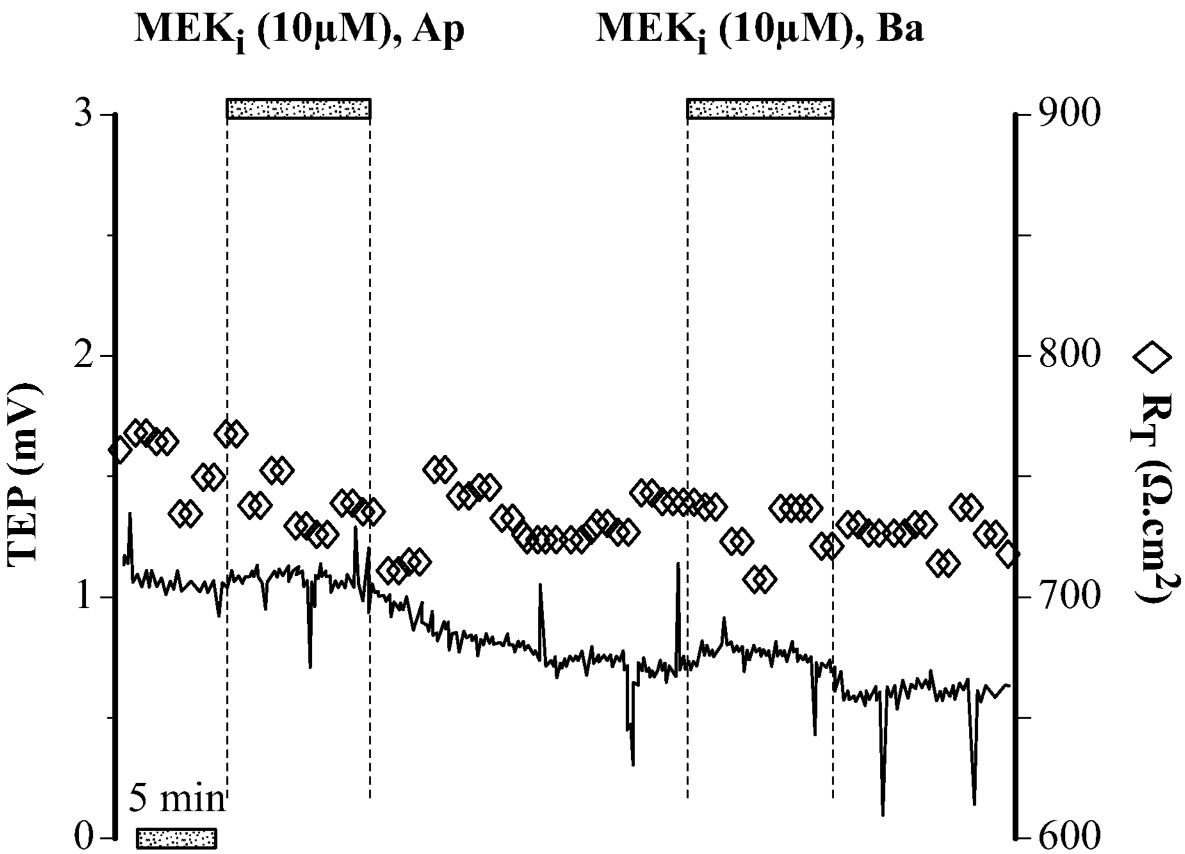
MEKi (10µM), Ap
MEKi (10µM), Ba
3
2
1
0
900
800
700
600
TEP (mV)
◇ RT (Ω.cm2)
5 min

FIG. 2A

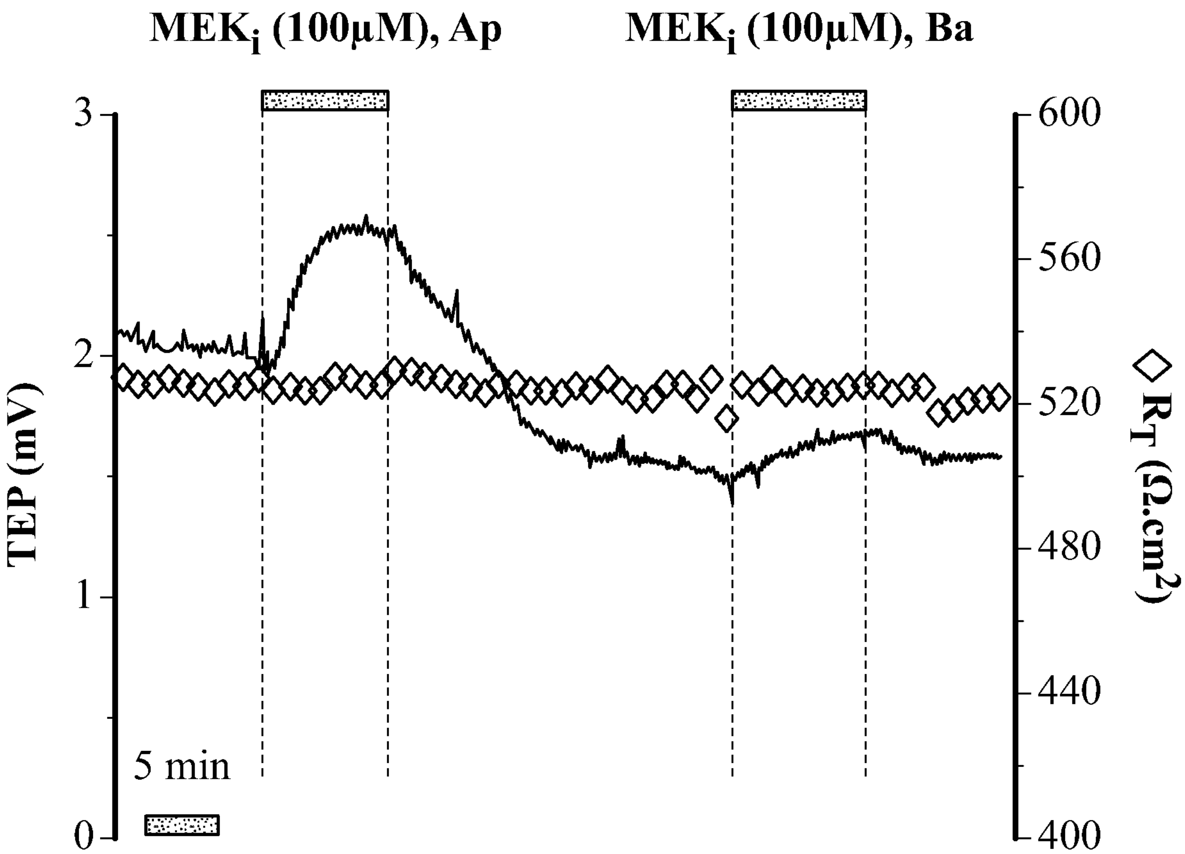

MEKi (100µM), Ap
MEKi (100µM), Ba
TEP (mV)
3
2
1
0
5 min
600
560
520
480
440
400
◇ RT (Ω.cm2)


*FIG. 2B*

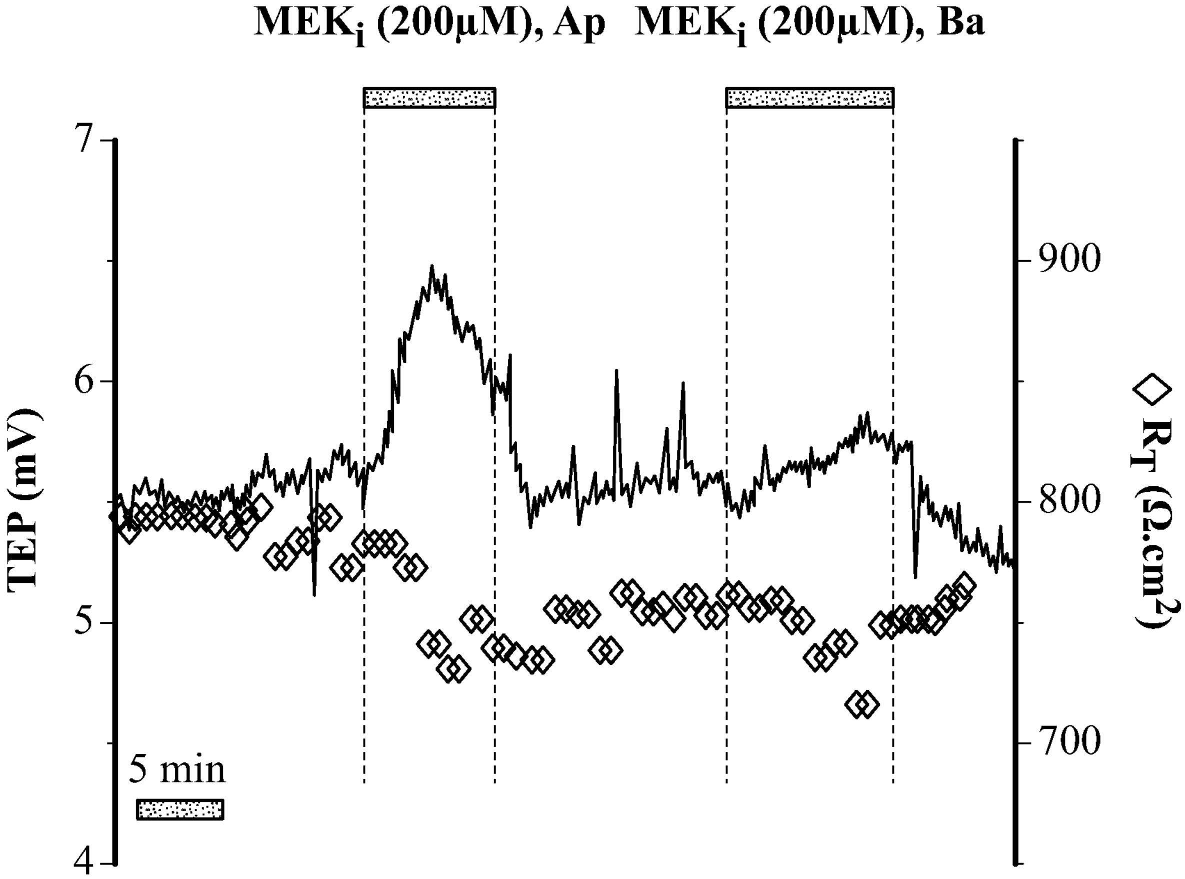
MEK$_i$ (200μM), Ap
MEK$_i$ (200μM), Ba
7
6
5
4
TEP (mV)
900
800
700
◇ R$_T$ (Ω.cm$^2$)
5 min

*FIG. 2C*

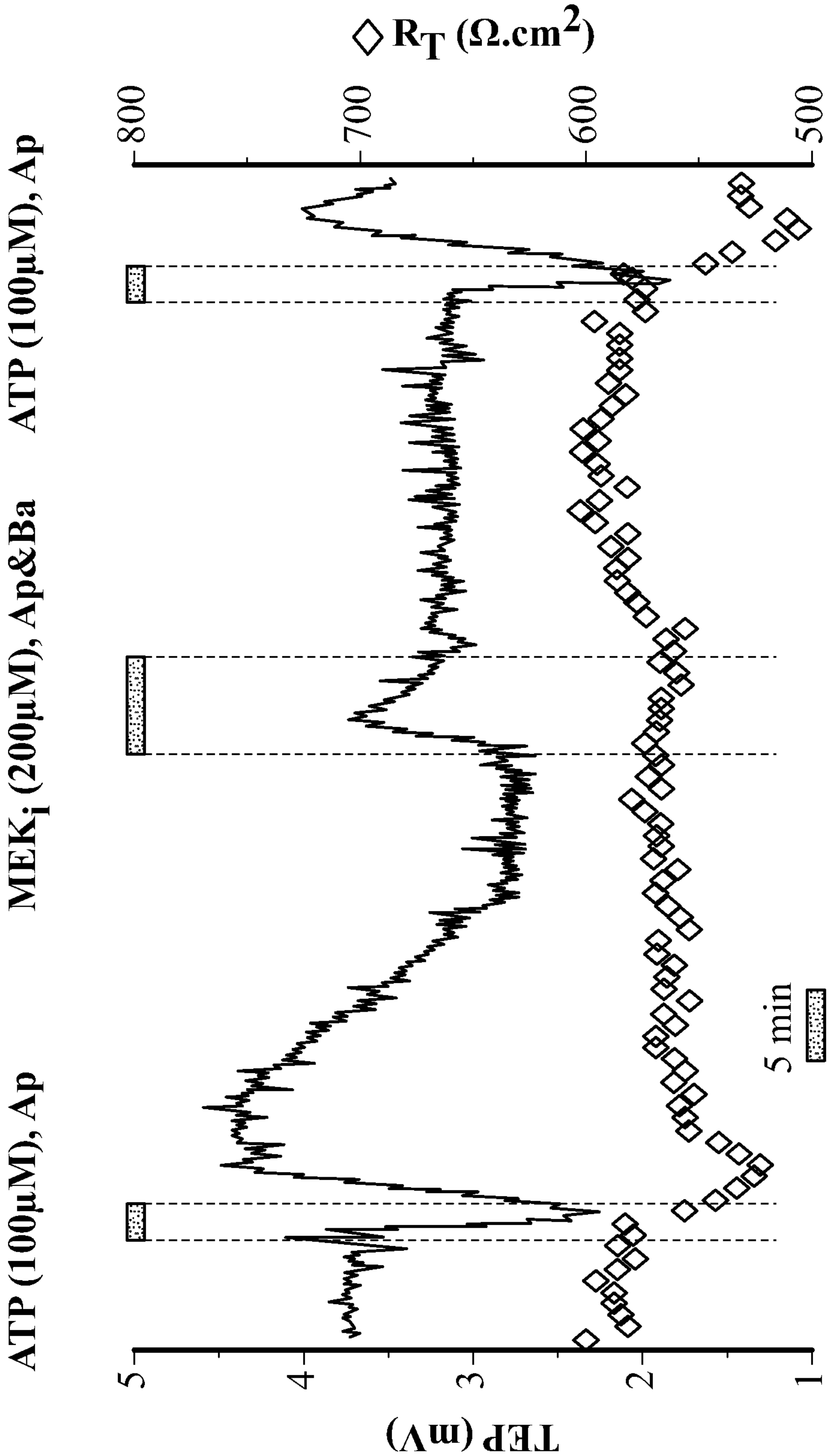
ATP (100µM), Ap
MEKi (200µM), Ap&Ba
ATP (100µM), Ap
◇ RT (Ω.cm2)
800
700
600
500
TEP (mV)
5
4
3
2
1
5 min

*FIG. 3*

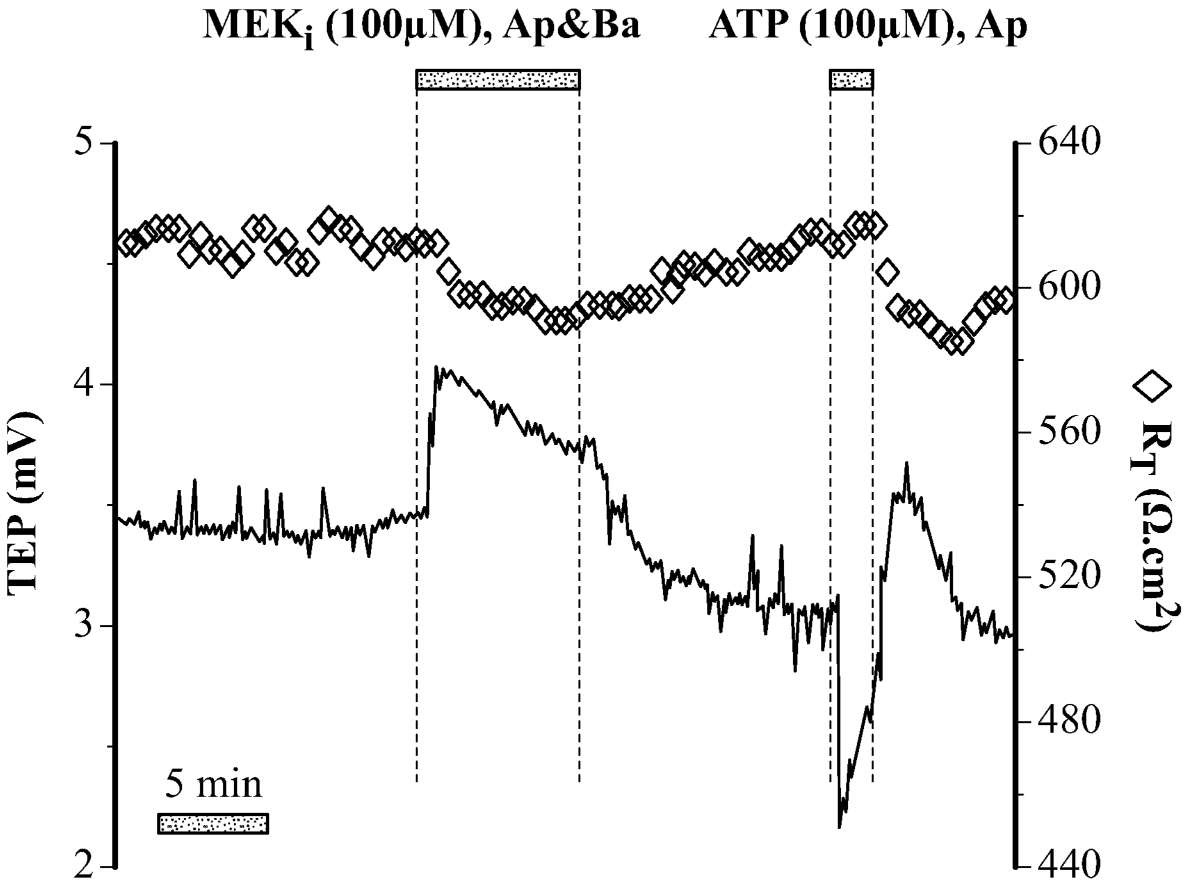

MEK$_i$ (100µM), Ap&Ba
ATP (100µM), Ap
5
4
3
2
TEP (mV)
640
600
560
520
480
440
◇ $R_T$ ($\Omega$.cm$^2$)
5 min


*FIG. 4A*

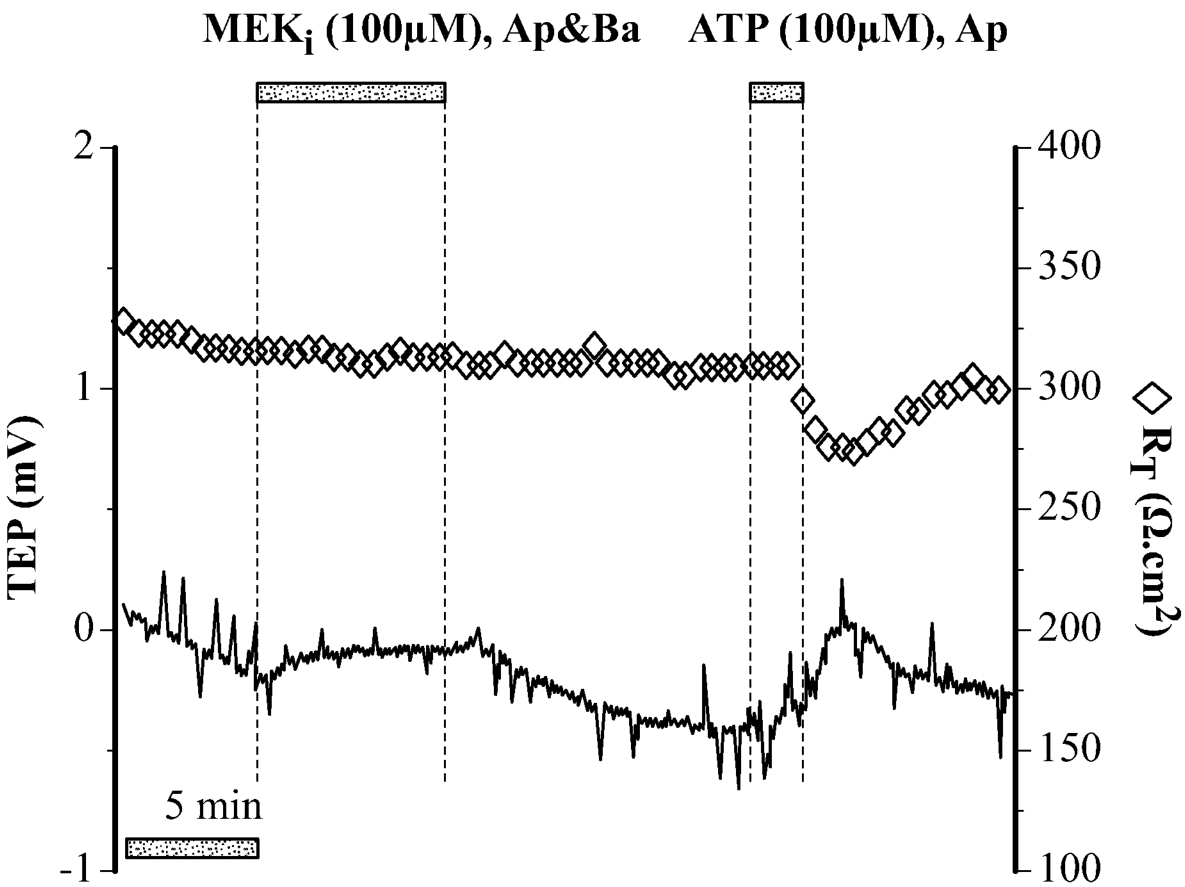
MEK$_i$ (100μM), Ap&Ba
ATP (100μM), Ap
TEP (mV)
◇ $R_T$ (Ω.cm$^2$)
5 min
2
1
0
-1
400
350
300
250
200
150
100

*FIG. 4B*

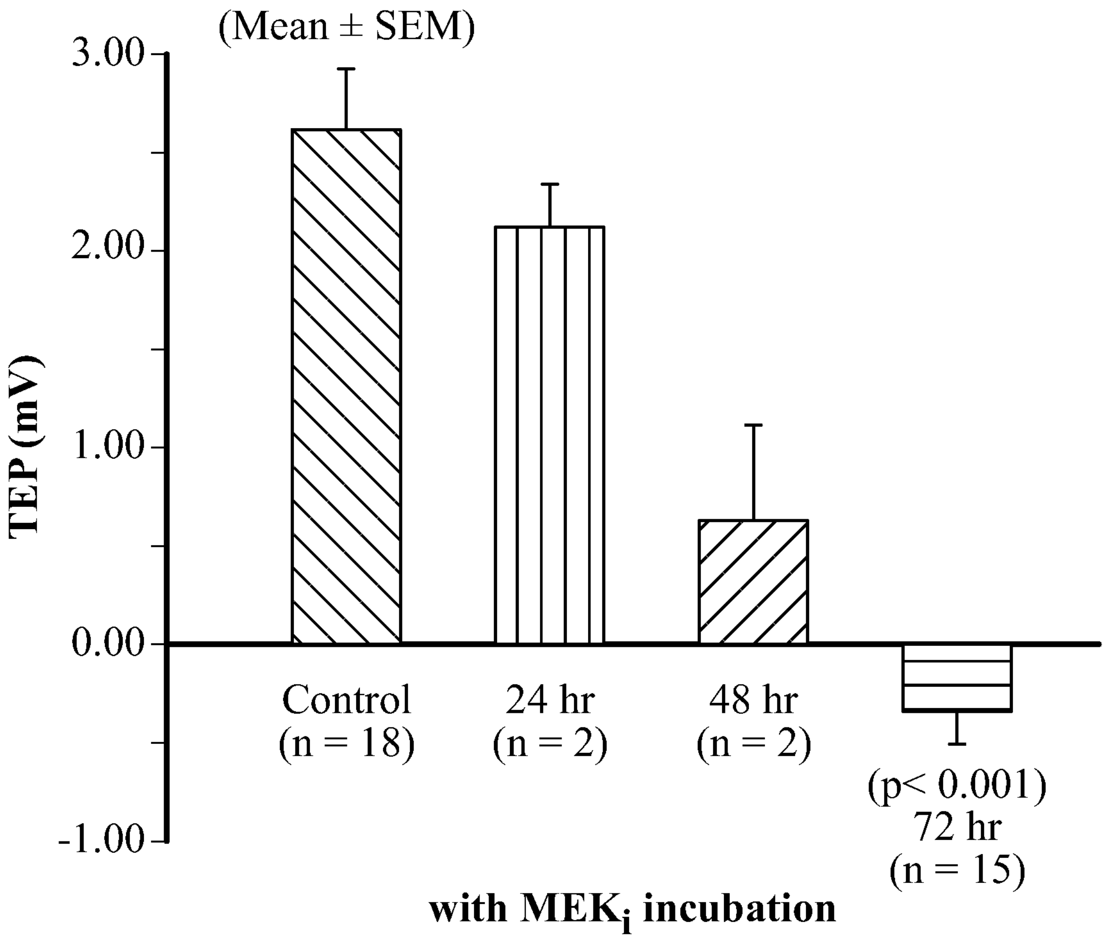
(Mean ± SEM)
3.00
2.00
1.00
0.00
-1.00
TEP (mV)
Control
(n = 18)
24 hr
(n = 2)
48 hr
(n = 2)
(p< 0.001)
72 hr
(n = 15)
with MEKi incubation

FIG. 5A

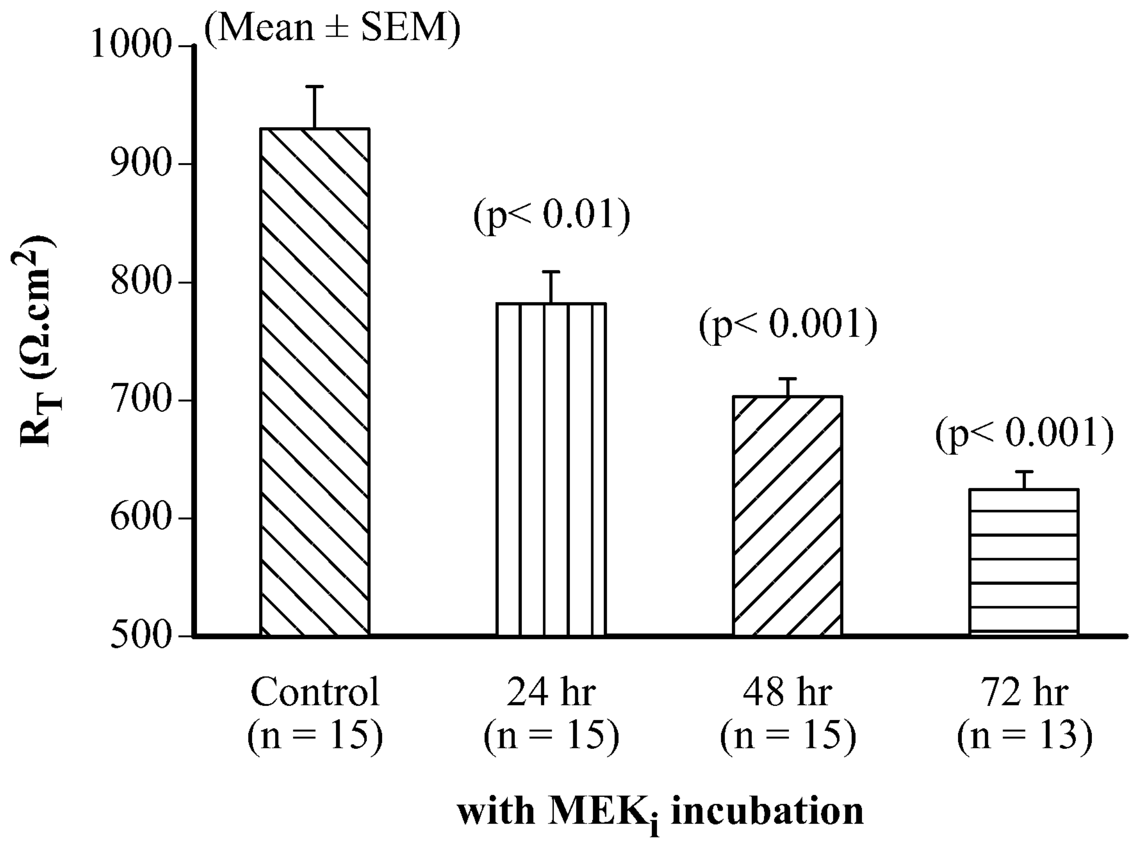
(Mean ± SEM)
1000
900
800
700
600
500
RT (Ω.cm2)
(p< 0.01)
(p< 0.001)
(p< 0.001)
Control
(n = 15)
24 hr
(n = 15)
48 hr
(n = 15)
72 hr
(n = 13)
with MEKi incubation

FIG. 5B

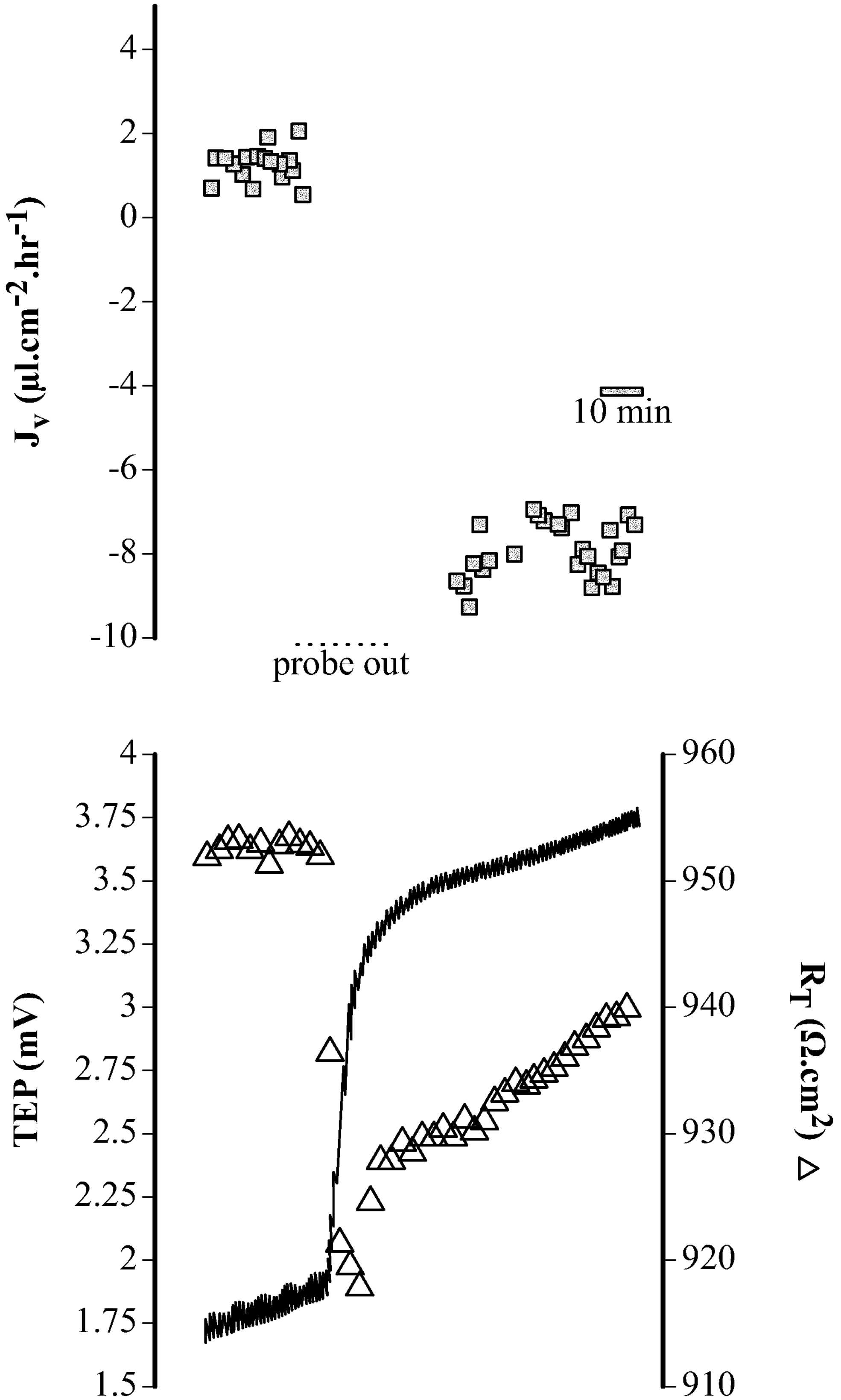
4
2
0
-2
-4
-6
-8
-10
$J_V$ (µl.cm$^{-2}$.hr$^{-1}$)
10 min
probe out
4
3.75
3.5
3.25
3
2.75
2.5
2.25
2
1.75
1.5
TEP (mV)
960
950
940
930
920
910
$R_T$ (Ω.cm$^2$) △

FIG. 6A

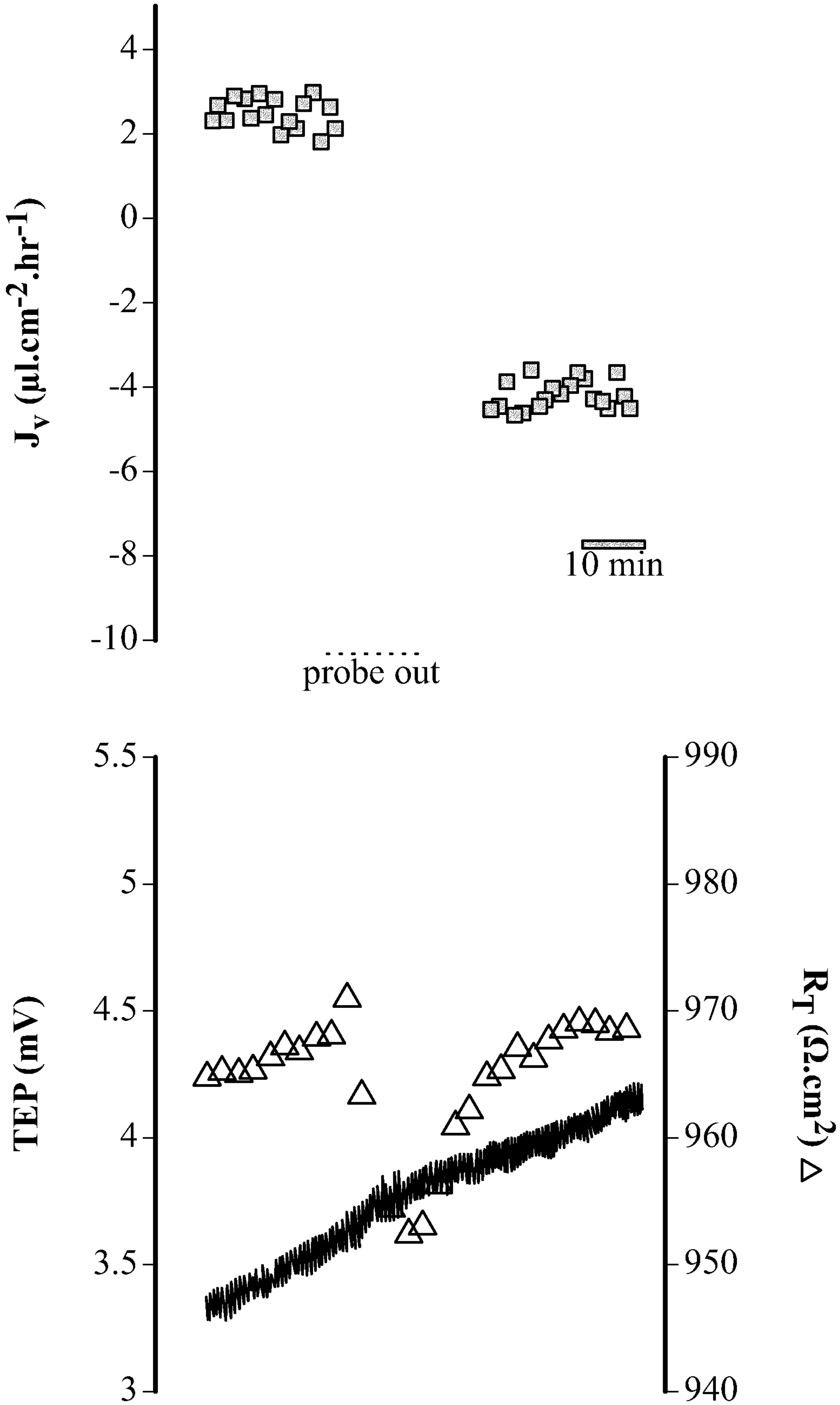
4
2
0
-2
-4
-6
-8
-10
$J_V$ (µl.cm$^{-2}$.hr$^{-1}$)
10 min
probe out
5.5
5
4.5
4
3.5
3
TEP (mV)
990
980
970
960
950
940
$R_T$ (Ω.cm$^2$) △

FIG. 6B

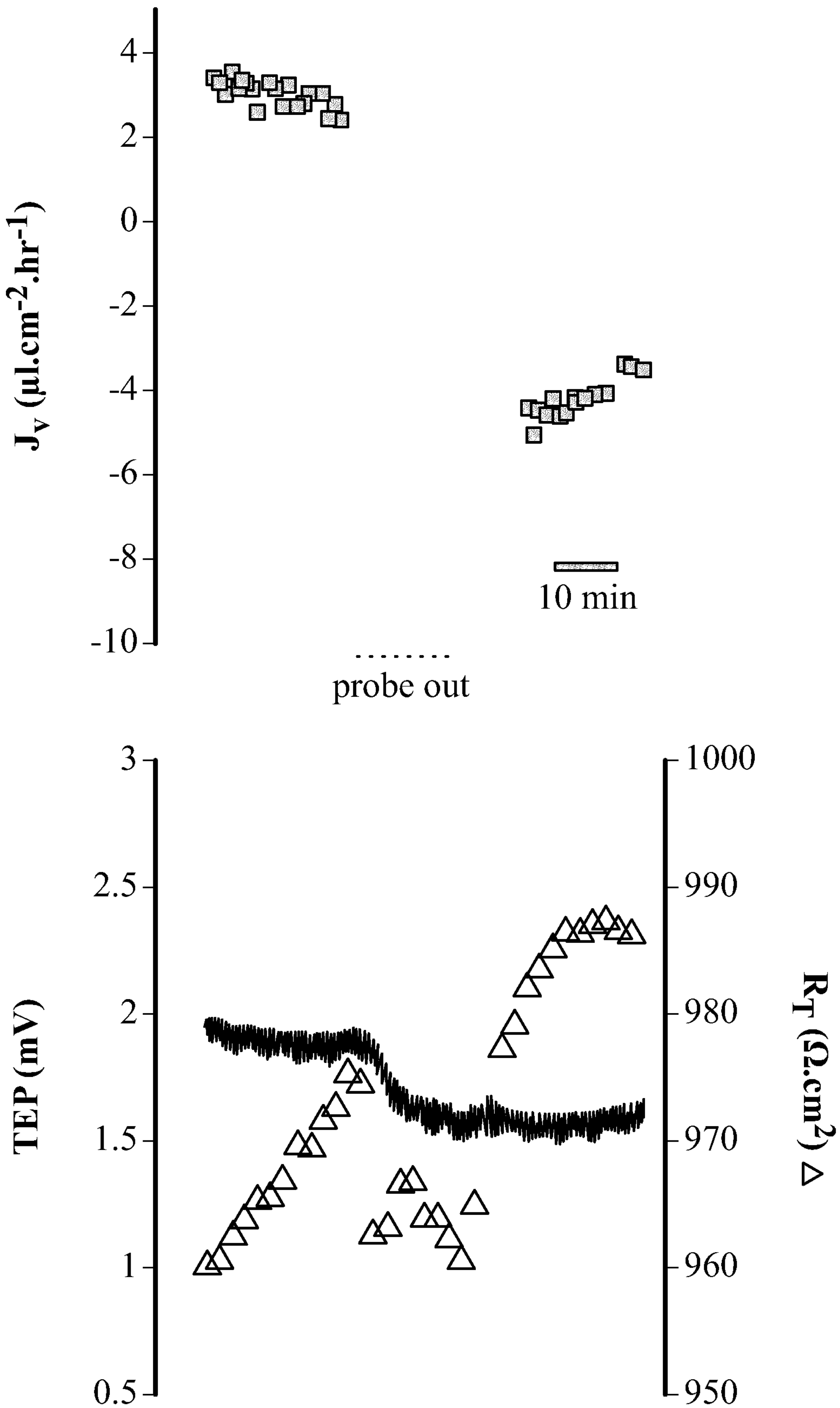
4
2
0
-2
-4
-6
-8
-10
$J_V$ (µl.cm$^{-2}$.hr$^{-1}$)
10 min
probe out
3
2.5
2
1.5
1
0.5
TEP (mV)
1000
990
980
970
960
950
$R_T$ (Ω.cm$^2$) △

FIG. 6C

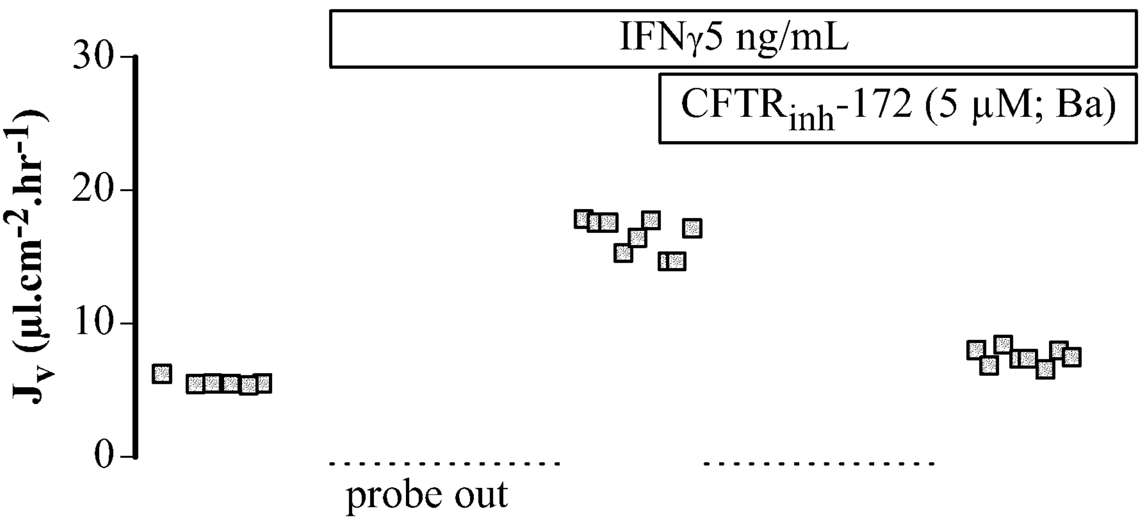


FIG. 7

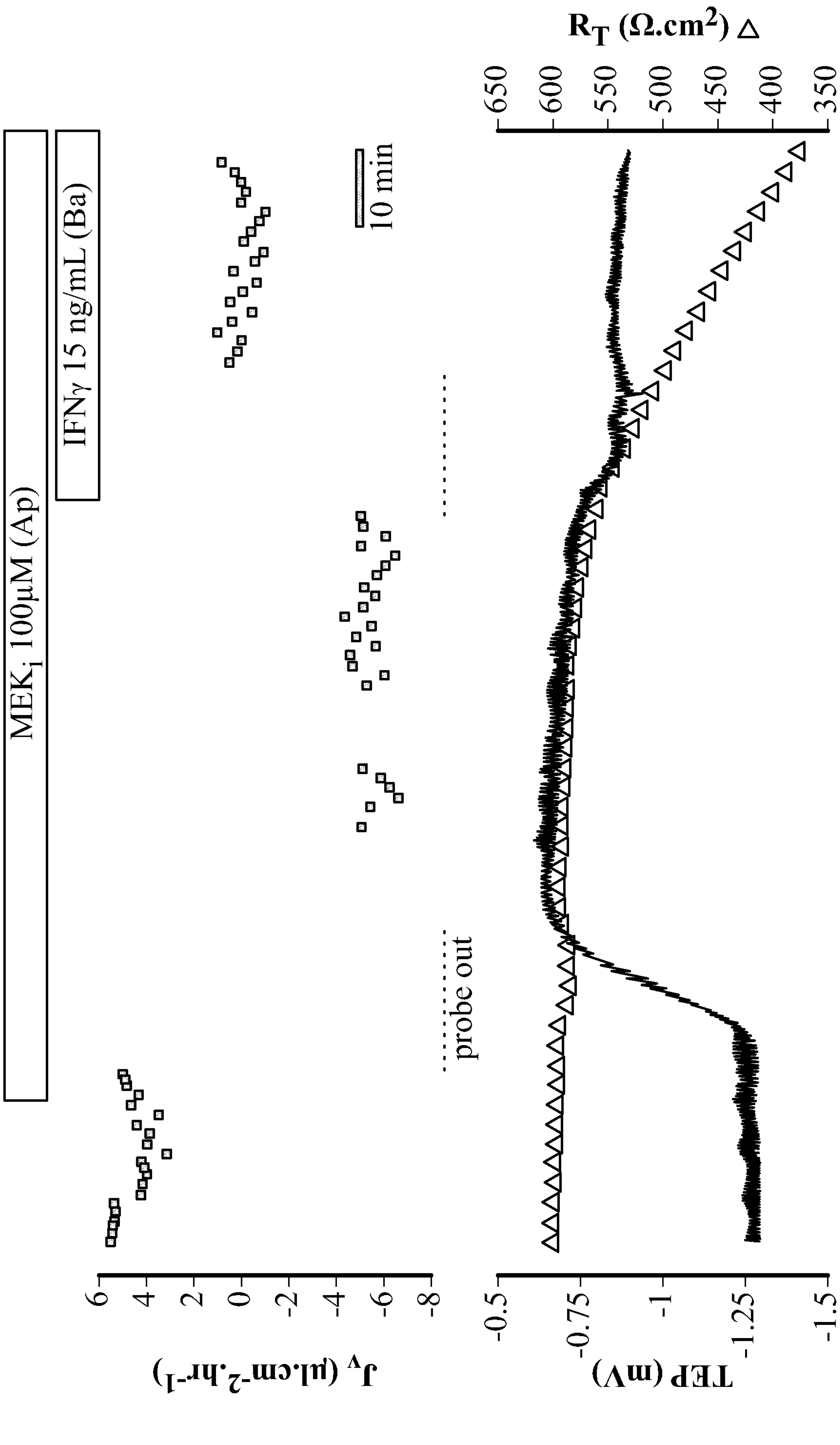

MEKi 100µM (Ap)
IFNγ 15 ng/mL (Ba)
10 min
probe out
Jv (µl.cm-2.hr-1)
6
4
2
0
-2
-4
-6
-8
TEP (mV)
-0.5
-0.75
-1
-1.25
-1.5
RT (Ω.cm2) △
650
600
550
500
450
400
350


FIG. 8

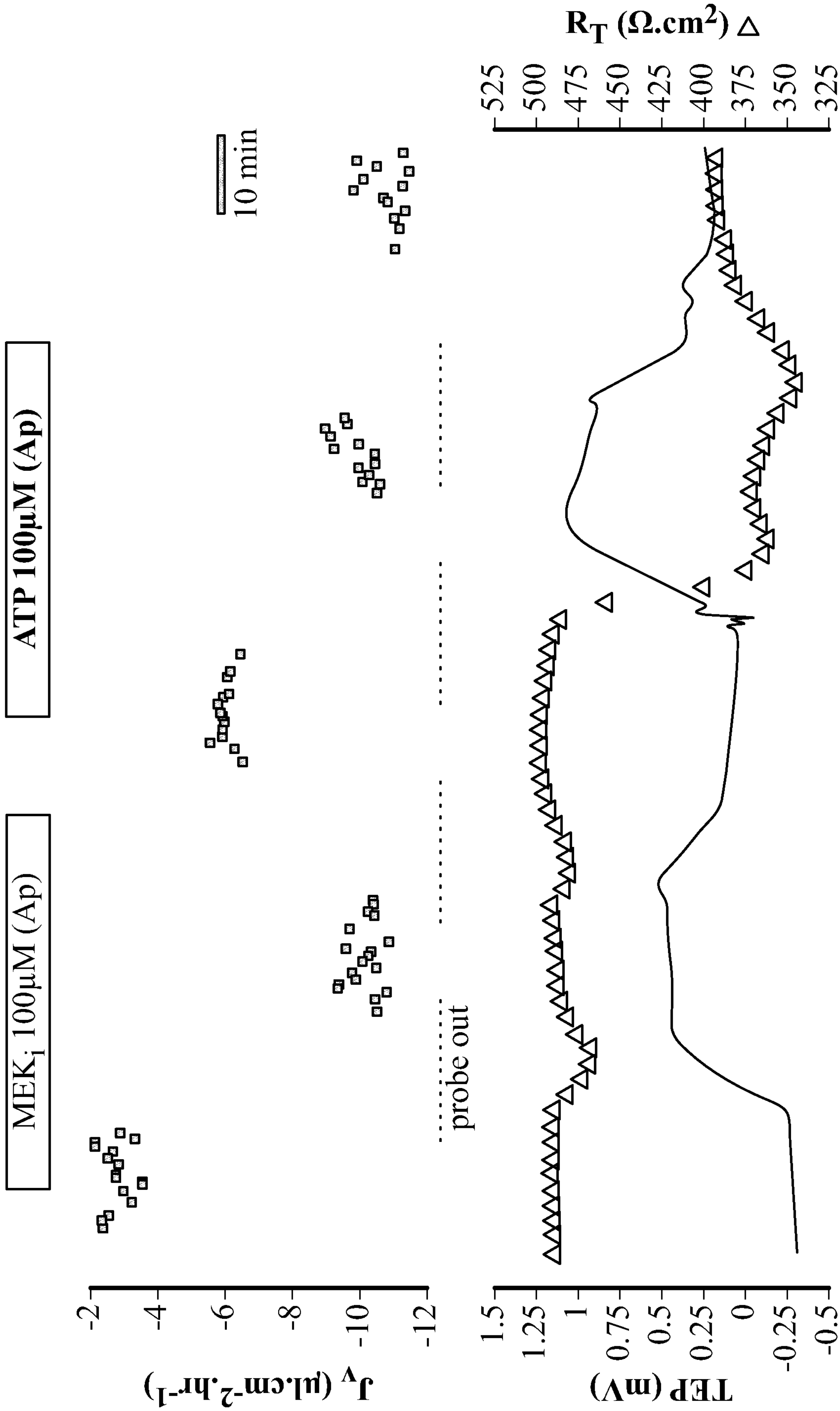
MEK$_i$ 100µM (Ap)
ATP 100µM (Ap)
10 min
probe out
$J_v$ (µl.cm$^{-2}$.hr$^{-1}$)
-2
-4
-6
-8
-10
-12
TEP (mV)
1.5
1.25
1
0.75
0.5
0.25
0
-0.25
-0.5
$R_T$ (Ω.cm$^2$) △
525
500
475
450
425
400
375
350
325

*FIG. 9*

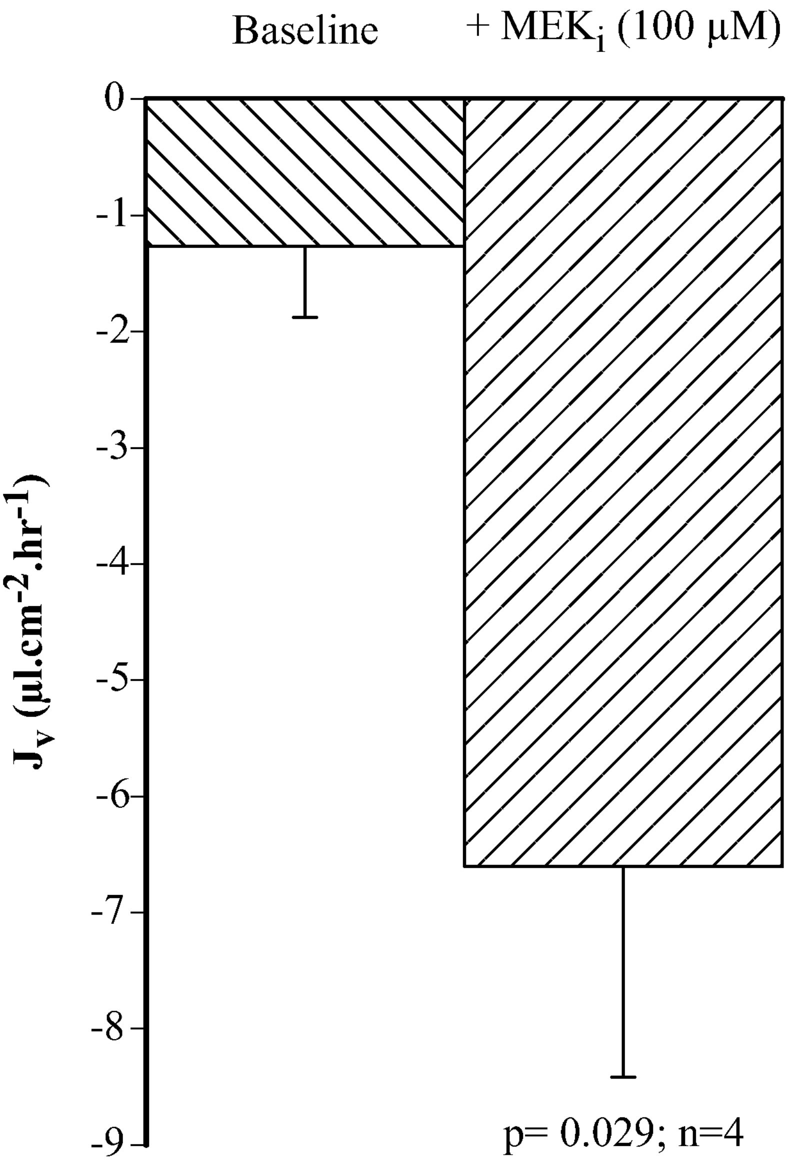


*FIG. 10A*

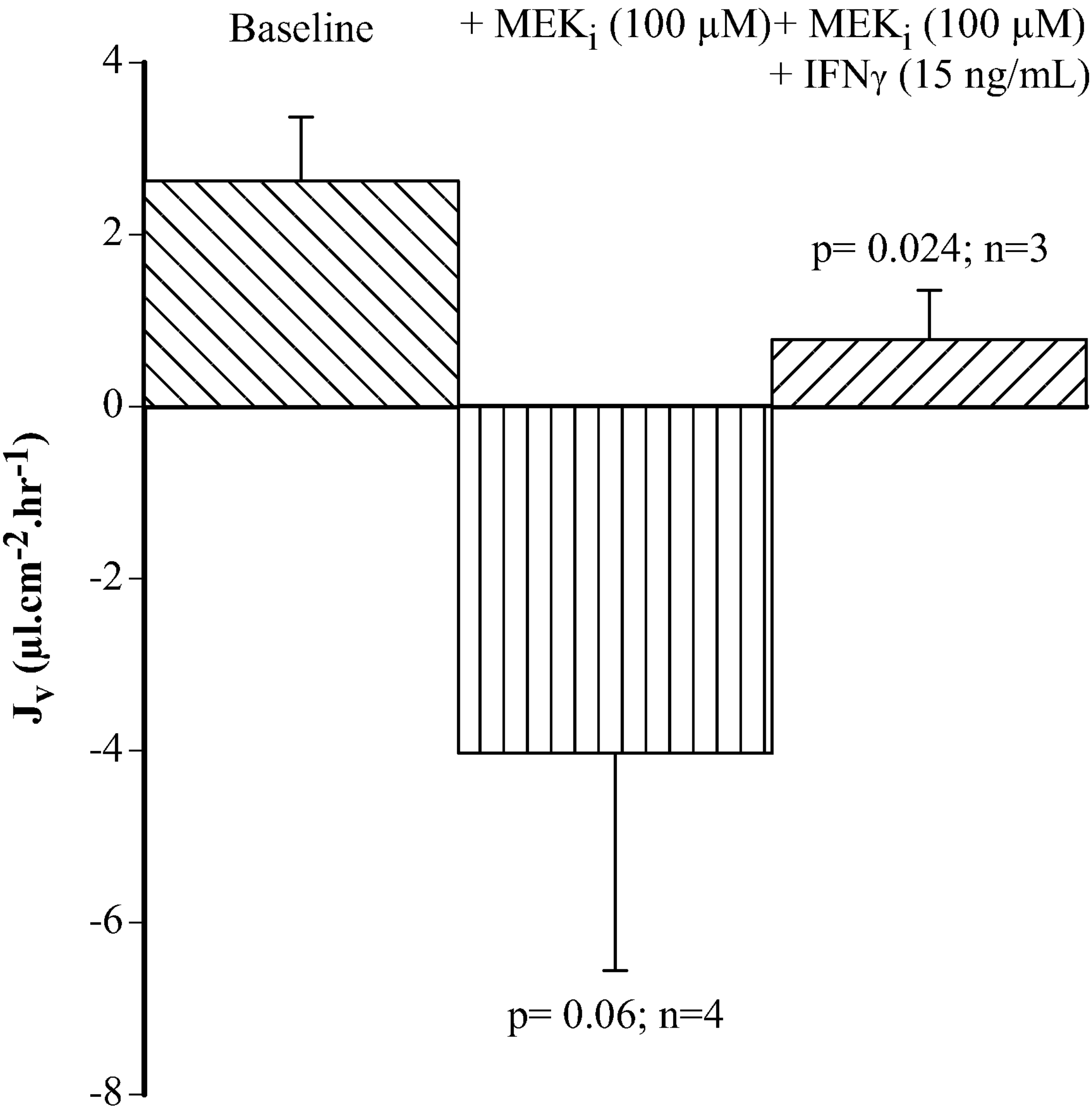
Baseline
+ MEKi (100 μM)
+ MEKi (100 μM)
+ IFNγ (15 ng/mL)
4
2
0
-2
-4
-6
-8
Jv (μl.cm-2.hr-1)
p= 0.024; n=3
p= 0.06; n=4

*FIG. 10B*

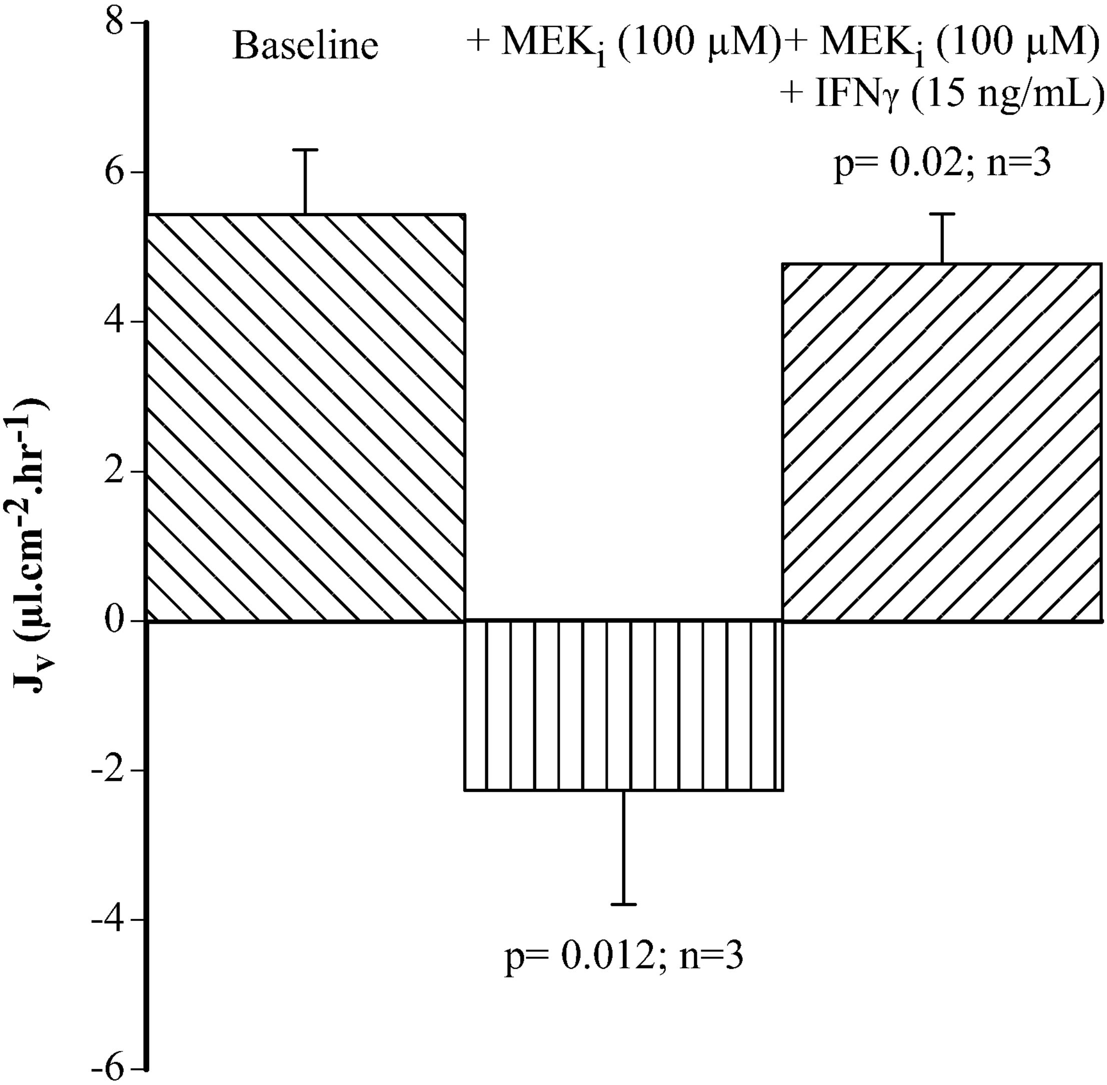
8
6
4
2
0
-2
-4
-6
$J_v$ (µl.cm$^{-2}$.hr$^{-1}$)
Baseline
+ MEK$_i$ (100 µM)
+ MEK$_i$ (100 µM)
+ IFNγ (15 ng/mL)
p= 0.02; n=3
p= 0.012; n=3

*FIG. 11A*

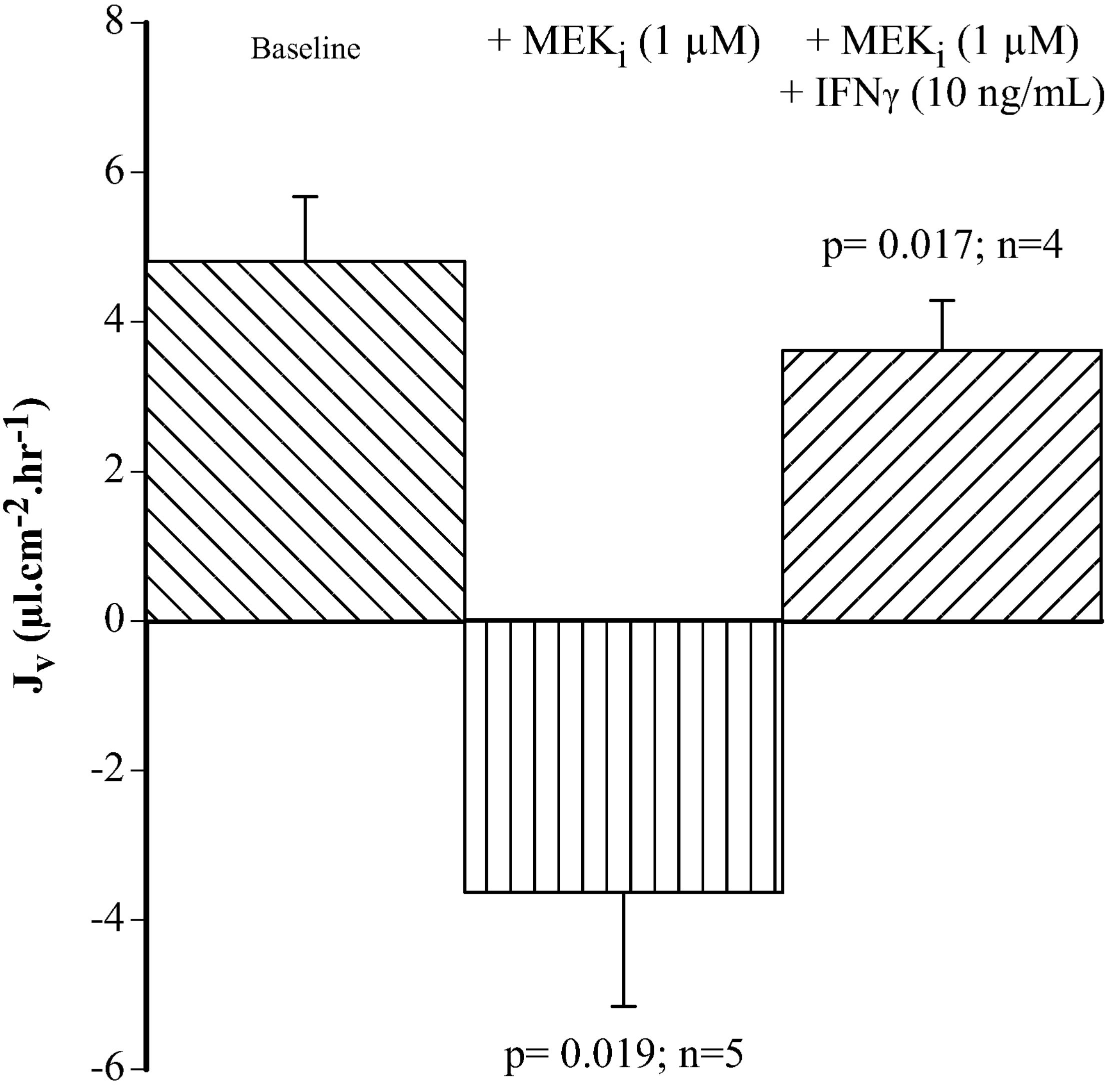
Baseline
+ MEKi (1 μM)
+ MEKi (1 μM) + IFNγ (10 ng/mL)
p= 0.017; n=4
p= 0.019; n=5
Jv (μl.cm-2.hr-1)
8
6
4
2
0
-2
-4
-6

*FIG. 11B*

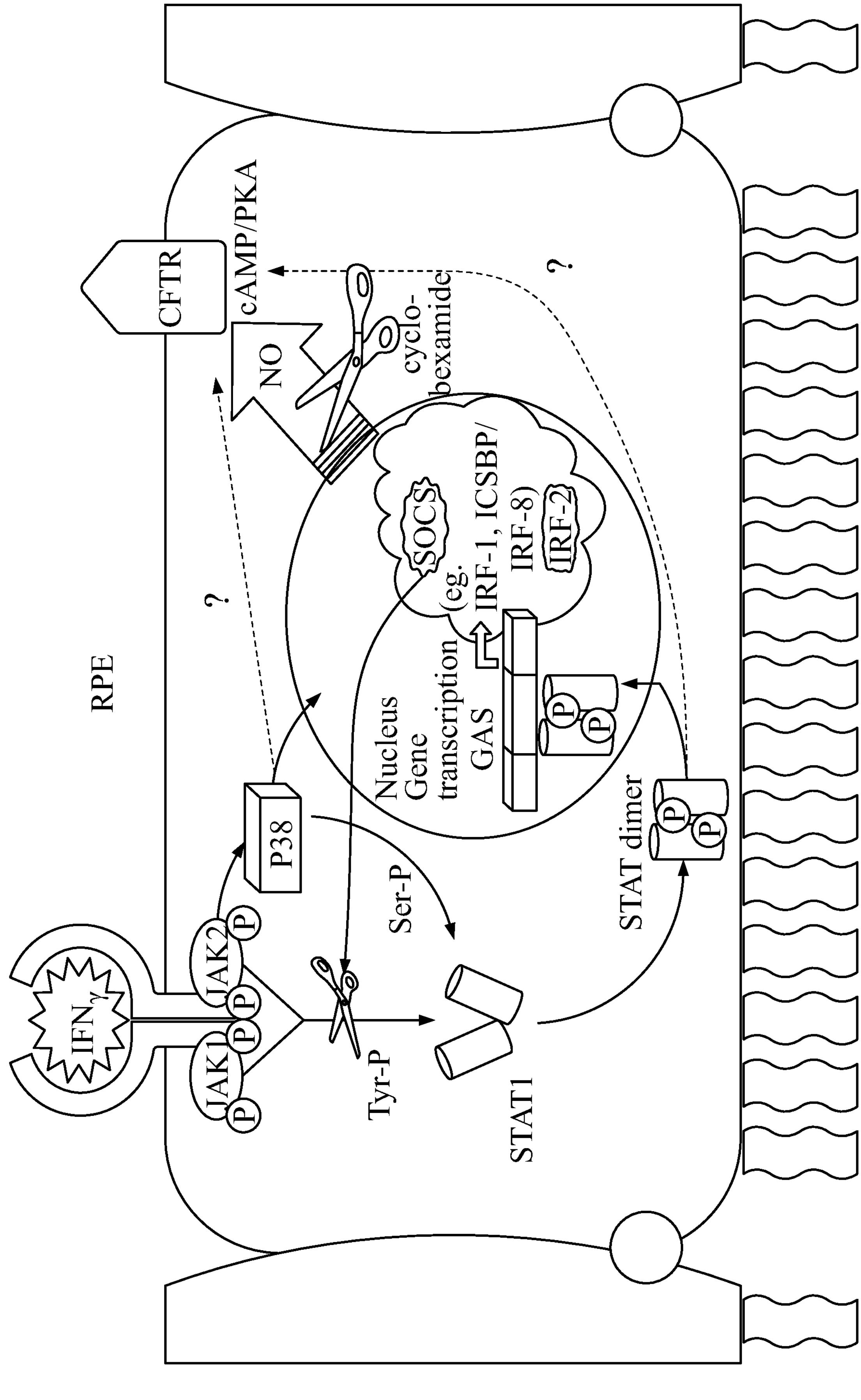
RPE
CFTR
cAMP/PKA
NO
cyclo-
bexamide
?
?
IFNγ
JAK1
JAK2
P
P38
Ser-P
Tyr-P
STAT1
STAT dimer
Nucleus
Gene
transcription
GAS
SOCS
(eg.
IRF-1, ICSBP/
IRF-8)
IRF-2

*FIG. 12*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010138377 A
- WO 2009153554 A
- WO 2009093009 A
- WO 2009013462 A
- WO 2009093013 A
- WO 2008020206 A
- WO 2008078086 A
- WO 2008120004 A
- WO 2008125820 A
- WO 2009093008 A
- WO 2009074827 A
- WO 2010108652 A
- WO 2010105110 A
- WO 2010105082 A
- WO 2009129246 A
- WO 2009018238 A
- WO 2009018233 A
- WO 2008089459 A
- US 20080255133 A
- US 20080058340 A
- WO 2008124085 A
- WO 2008076415 A
- WO 2008021389 A
- WO 2010051935 A
- WO 2010051933 A
- WO 2009129938 A
- WO 2009021887 A
- WO 2008101840 A
- US 20090275606 A
- US 20090246198 A
- WO 2008055236 A
- WO 2010003025 A
- WO 2010003022 A
- WO 2007096259 A
- WO 2008067481 A
- WO 2008024724 A
- WO 2008024725 A
- WO 20100145197 A
- EP 1883395 A2
- WO 2010002418 A
- US 6413245 B


### Non-patent literature cited in the description


- **TRUJILLO.** *Expert Opin Ther Patents,* 2011, vol. 21, 1045-69
- **MESSERSMITH et al.** *Clin Adv Hematol Oncol,* 2006, vol. 4, 831-36
- **HAURA et al.** *Clin Cancer Res,* 2010, vol. 16, 2450-57
- **RENOUF et al.** *JCO,* 2011, vol. 41, 5851
- **FREMIN et al.** *J Hemotol Oncol,* 2010, vol. 3, 8-11
- **GIRARD et al.** *meeting,* 08 June 2012
- **RINEHART et al.** *J Clin Oncol,* 2004, vol. 22, 4456-62
- **WANG et al.** *BBA Mol Cell Res,* 2007, vol. 1773, 1248-55
- **STARK, G. et al.** *Annu Rev Biochem,* 1998, vol. 67, 227-264
- **RAMANA, C. et al.** *Trends Immunol,* 2002, vol. 23, 96-101
- **VAN BOXEL-DEZAIRE, A. et al.** *Curr Top Microbiol Immunol,* 2007, vol. 316, 119-154
- **BACH, E. et al.** *Annu Rev Immunol,* 1997, vol. 15, 563-591
- **HEMMI, S. et al.** *Cell,* 1994, vol. 76, 803-810
- **SOH, J. et al.** *Cell,* 1994, vol. 76, 793-802
- **THIPPHAWONG.** *Adv Drug Del Rev,* 2006, vol. 58, 1089-1105
- **GALIETTA et al.** *Proc Am Thorac Soc,* 2004, vol. 1, 62-65
- **YUAN et al.** *Vaccine,* 2008, vol. 26, 3322-31
- **FERRIS FL et al.** *Am J Ophthalmol,* 1982, vol. 94, 91-96
- Lighthouse Distance Visual Acuity Test charts
- **SAMUELS et al.** *J Neurophysiol,* 2010, vol. 104, 391-402
- **WU et al.** *Mol Vis,* 2004, vol. 10, 650-54
- **MAMINISHKIS, A. et al.** *Invest Ophthalmol Vis Sci,* 2006, vol. 47, 3612-3624
- **VOLOBOUEVA, L.A. et al.** *Invest Ophthalmol Vis Sci,* 2005, vol. 46, 4302-4310
- **SHI, G. et al.** *Invest Ophthalmol Vis Sci,* 2008
- **LI, R. et al.** *Invest Ophthalmol Vis Sci,* 2007, vol. 48, 5722-5732
- **QUINN, R.H. et al.** *Invest Ophthalmol Vis Sci,* 1992, vol. 33, 3513-3527
- **JOSEPH D.P. et al.** *J Physiol,* 1991, vol. 435, 439-463
- **SHI, G. et al.** *Invest Ophthalmol Vis Sci,* 2008, vol. 49, 4620-4630

- **EDELMAN, J.L. et al.** *Invest Ophthalmol Vis Sci,* 1991, vol. 32, 3033-3040
- **JIANG, C. et al.** *Science,* 1993, vol. 262, 424-427
- **MAMINISHKIS, A. et al.** *Invest Ophthalmol Vis Sci,* 2002, vol. 43, 3555-3566
- **PETERSEN et al.** *J Neurosci,* 1997, vol. 17, 2324-37
- **LI et al.** *Am J Physiol Cell Physiol,* 2009, vol. 297, C1452-65